(19) Europäisches Patentamt
European Patent Office
Office européen des brevets

(11) Publication number: **0 630 904 A1**

(12) # EUROPEAN PATENT APPLICATION published in accordance with Art. 158(3) EPC

(21) Application number: **94908116.0**

(51) Int. Cl.$^5$: **C07H 17/02**

(22) Date of filing: **17.08.93**

(86) International application number: **PCT/JP93/01151**

(87) International publication number: **WO 94/04547 (03.03.94 94/06)**

(30) Priority: **26.08.92 JP 227526/92**
**07.10.92 JP 268822/92**
**27.11.92 JP 319034/92**
**17.03.93 JP 57478/93**
**04.06.93 JP 134861/93**

(43) Date of publication of application: **28.12.94 Bulletin 94/52**

(84) Designated Contracting States: **DE FR GB**

(71) Applicant: **JAPAN TOBACCO INC.**
**12-62, Higashishinagawa 4-chome,**
**Shinagawa-ku,**
**Tokyo 140 (JP)**

(72) Inventor: **SHIBAGAKI, Makoto, Japan Tobacco Inc.**
**Life ScienceRes. Lab.,**
**6-2, Umegaoka,**
**Midori-ku**
**Yokohama-shi,**
**Kanagawa-ken 227 (JP)**
Inventor: **TAKAHASHI, Kyoko, Japan Tobacco Inc.**
**Life Science Res. Lab.,**
**6-2, Umegaoka,**
**Midori-ku**
**Yokohama-shi,**
**Kanagawa-ken 227 (JP)**
Inventor: **KUNO, Hideyuki, Japan Tobacco Inc.**
**Life Science Res. Lab.,**
**6-2, Umegaoka,**
**Midori-ku**
**Yokohama-shi,**
**Kanagawa-ken 227 (JP)**
Inventor: **MATSUSHITA, Hajime, Japan Tobacco Inc.**
**Life Science Res. Lab.,**
**6-2, Umegaoka,**
**Midori-ku**
**Yokohama-shi,**
**Kanagawa-ken 227 (JP)**

(74) Representative: **Ruffles, Graham Keith**
**MARKS & CLERK,**
**57-60 Lincoln's Inn Fields**
**London WC2A 3LS (GB)**

(54) **NOVEL NICOTINE DERIVATIVE.**

(57) A novel nicotine derivative represented by general formula (1) wherein R represents a hexose or a dihexose; and (a) represents (S)-(-)-(3)-(1-methyl-2-pyrrolidinyl)-2-pyridyl, (R)-(+)-3-(1-methyl-2-pyrrolidinyl)-2-pyridyl, (S)-(-)-5-(1-methyl-2-pyrrolidinyl)-2-pyridyl, or (R)-(+)-5-(1-methyl-2-pyrrolidinyl)-2-pyridyl.

EP 0 630 904 A1

[Technical Field]

The present invention relates to a novel and an optically active nicotine derivative, and particularly to a nicotine derivatives containing a glycosyl group represented by the following formula (1).

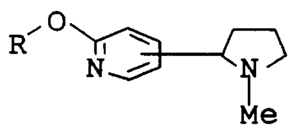

(1)

Wherein R represents a hexose or a dihexose. Further,

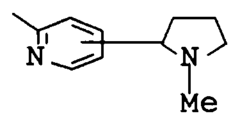

is an (S)-(-)-3-(1-methyl-2-pyrrolidinyl)-2-pyridyl group, an (R)-(+)-3-(1-methyl-2-pyrrolidinyl)-2-pyridyl group, an (S)-(-)-5-(1-methyl-2-pyrrolidinyl)-2-pyridyl group, or an (R)-(+)-5-(1-methyl-2-pyrrolidinyl)-2-pyridyl group.

[Background Art]

Nicotine is an alkaloid obtained as a by-product in the tobacco industry. A useful derivative of nicotine is also known to have an effect of improving the flavor and taste of tobacco (for Example, Jpn. Pat. Nos. 1371806 and 1456909).

As a physiological activity of nicotine, for example, an autonomic nervous regulatory activity, a circulatory-system regulatory activity such as vasopressor activity, or a digestive organ regulatory activity such as gastrointestinal activation (for example, Seikagaku, 57, 447 (1985), J. Pharm. Experi. Ther., 222, 463 (1983), Br. J. Pharmac., 79, 869 (1983)) are known. Further, alkoxy nicotine is known to have an antimicrobial activity and an antibacterial activity (U.S. Patent Serial No. 3,644,176). As mentioned above, nicotine and a nicotine derivative have a variety of physiological activities, and they are expected to be useful as a pharmaceutical agent. In addition, a sulfate of nicotine is used as an agricultural insecticide, and application of nicotine or nicotine derivative to the agricultural field is also expected.

However, since nicotine has toxicity by itself and its volatility is high, application of nicotine to the above-mentioned individual fields or practical use of nicotine in those fields has not yet been achieved. Accordingly, there is a demand for a nicotine derivative having its useful properties and capable of solving a problem such as toxicity.

The present invention has been made under the above-mentioned circumstances and has an object to provide a novel nicotine derivative which is useful as an agent for improving the flavor and taste of tobacco, which can be expected to be used as a physiologically active substance.

[Disclosure of Invention]

The object of the present invention can be achieved by a novel nicotine derivative represented by the following formula (1)

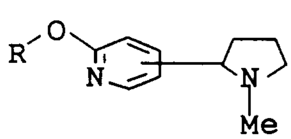

(1)

wherein R and a group represented by

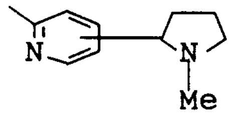

are the same as defined above.

Hereinbelow, the present invention will be explained in detail.

The novel nicotine derivative of the present invention is a compound represented by the following formula (1).

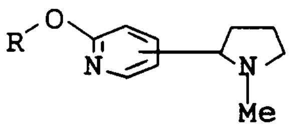

(1)

wherein, R and a group represented by

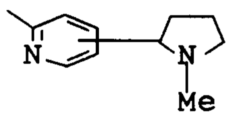

are the same defined as above.

In the compound (1) of the present invention, R represents a monosaccharide or disaccharide of a general hexose. To be more specific, a hexose such as glucose, galactose, and mannose; an aminohexose such as glucosamine, galactosamine, and mannosamine; a dihexose such as lactose, maltose, and cellobiose; or an aminodihexose such as lactosamine. In the present invention, it is preferred to use glucose or galactose which is easily obtained, inexpensive, and relatively easier to convert into a glycosyl donor.

In the compound (1) of the present invention, an aglycone part: [a (1-methyl-2-pyrrolidinyl)-2-pyridyl group] represented by the following formula (2), specifically an (S)-(-)-3-(1-methyl-2-pyrrolidinyl)-2-pyridyl group, an (R)-(+)-3-(1-methyl-2-pyrrolidinyl)-2-pyridyl group, an (S)-(-)-5-(1-methyl-2-pyrrolidinyl)-2-pyridyl group, and an
(R)-(+)-5-(1-methyl-2-pyrrolidinyl)-2-pyridyl group.

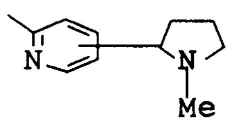

(2)

Hereinafter, the feature of the compound (1) of the present invention will be described.

The compound (1) of the present invention is characterized in that the compound has a (1-methyl-2-pyrrolidinyl)-2-pyridyl group represented as an aglycone by the above formula (2) and a monosaccharide or a disaccharide of a general hexose as a glycosyl part, both of which are bound with O-glycoside.

The compound (1) of the present invention is preferable that in a glycosyl part, it contain glucose and galactose whose raw materials are easily obtained and whose derivatives can be easily synthesized. Further, in a view of easy preparation, a compound containing a $\beta$-O-glycoside bond is preferred. Hence, in the present invention, eight compounds represented by the following formula from (1a) to (1h) are particularly preferred.

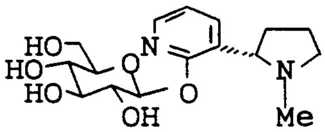

(1a)

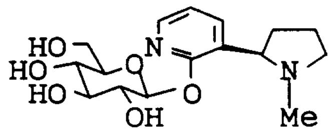

(1b)

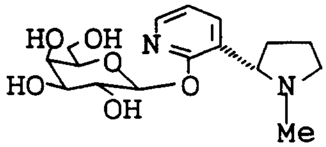

(1c)

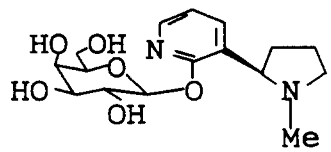

(1d)

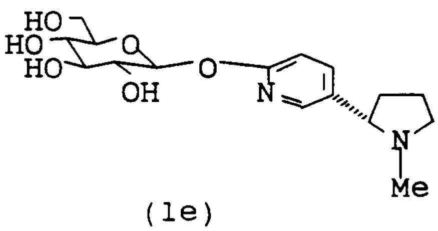

(1e)

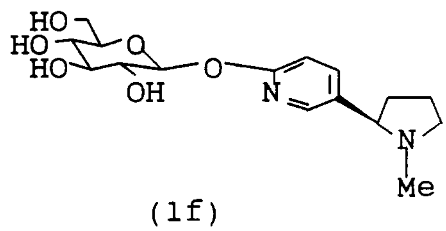

(1f)

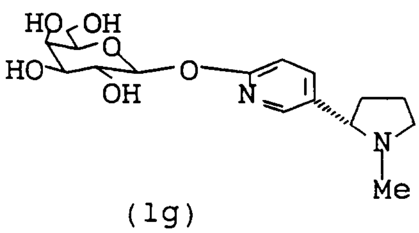

(1g)

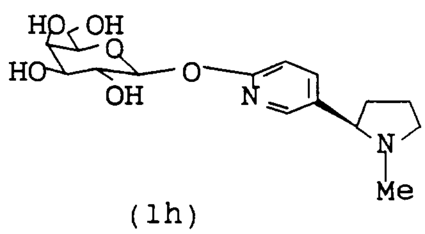

(1h)

The feature of the preferred compounds (1a) to (1h) of the present invention will be described.

1. Compound (1a)

The compound (1a) is characterized in that it has an (S)-(-)-3-1-methyl-2-pyrrolidinyl)-2-pyridyl group as an aglycone and glucose as a glycosyl part, both of which are bound with β-O-glycoside.

2. Compound (1b)

The compound (1b) is characterized in that it has an (R)-(+)-3-1-methyl-2-pyrrolidinyl)-2-pyridyl group as an aglycone and glucose as a glycosyl part, both of which are bound with a β-O-glycoside.

3. Compound (1c)

The compound (1c) is characterized in that it has an (S)-(-)-3-1-methyl-2-pyrrolidinyl)-2-pyridyl group as an aglycone and galactose as a glycosyl part, both of which are bound with a β-O-glycoside.

4. Compound (1d)

The compound (1d) is characterized in that it has an (R)-(+)-3-1-methyl-2-pyrrolidinyl)-2-pyridyl group as an aglycone and galactose as a glycosyl part, both of which are bound with a β-O-glycoside.

5. Compound (1e)

The compound (1e) is characterized in that it has an (S)-(-)-5-1-methyl-2-pyrrolidinyl)-2-pyridyl group as an aglycone and glucose as a glycosyl part, both of which are bound with β-O-glycoside.

6. Compound (1f)

The compound (1f) is characterized in that it has an (R)-(+)-5-1-methyl-2-pyrrolidinyl)-2-pyridyl group as an aglycone and glucose as a glycosyl part, both of which are bound with β-O-glycoside.

7. Compound (1g)

The compound (1g) is characterized in that it has an (S)-(-)-5-1-methyl-2-pyrrolidinyl)-2-pyridyl group as an aglycone and galactose as a glycosyl part, both of which are bound with β-O-glycoside.

8. Compound (1h)

The compound (1h) is characterized in that it has an (R)-(+)-5-1-methyl-2-pyrrolidinyl)-2-pyridyl group as an aglycone and galactose as a glycosyl part, both of which are bound with β-O-glycoside.

Then, a method for producing the compound (1) of the present invention will be described.

The compound (1) of the present invention can be prepared by reacting a glycoside derivative (3) whose reduced end is activated by halogenation and the like with a nicotine derivative represented by a general formula (4), and by deprotecting the obtained compound represented by a general formula (5) as shown in the following reaction.

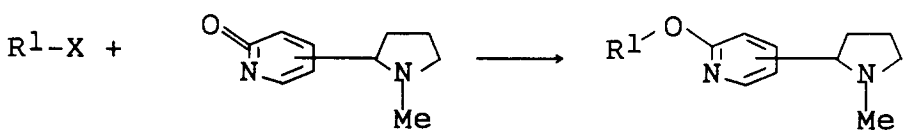

(3) (4) (5)

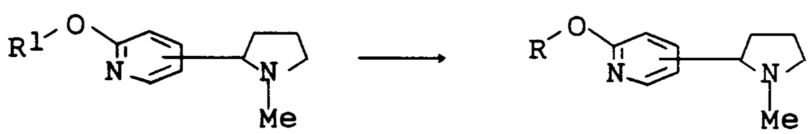

(5) (1)

wherein R and a group

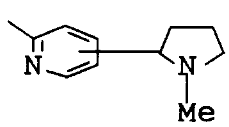

are the same as defined above. $R^1$ represents a glycosidic residue protected by an acyl group, a benzyl group, or the like and X is an active group of halogen or the like. Further, the compound (4) is
(S)-(-)-3-(1-methyl-2-pyrrolidinyl)-2-pyridone (4a),
(R)-(+)-3-(1-methyl-2-pyrrolidinyl)-2-pyridone (4b),
(S)-(-)-5-(1-methyl-2-pyrrolidinyl)-2-pyridone (4c), and
(R)-(+)-5-(1-methyl-2-pyrrolidinyl)-2-pyridone (4d).

A glycosyl donor, the compound (3) can be easily prepared by halogenation of a glycosyl derivative protected appropriately. As a halogenation agent which can be used in the present invention, a hydrobromic acid - acetic acid solution or carbon tetrachloride - tris(dimethylamino)phosphine, or the like is mentioned.

The glycosyl derivative protected appropriately is a hexose, an amino hexose, or a dihexose, whose hydroxyl groups, except a reduced end, are protected by an acyl group such as an acetyl group, or an aralkyl group such as a benzyl group, and whose reduced end is not protected or protected by an acyl group which can be converted into an active group. Specifically, glucose, galactose, mannose, glucosamine, galactosamine, mannosamine, lactose, maltose, cellobiose, or the like is mentioned. More specifically, 1,2,3,4,6-penta-O-acetylglucopyranoside, 2,3,4,6-tetra-O-acetylglucopyranose, or the like is mentioned.

As the compound (3), for example, 2,3,4,6-tetra-O-acetylglucopyranosyl bromide (3a) or 2,3,4,6-tetra-O-acetylgalactopyranosyl bromide (3b) is mentioned. The compound (3a) can be obtained by a method described in Nature, 165, 369 (1950). Further, the compound (3b) can be prepared by dissolving commercially available 1,2,3,4,6-penta-O-acetylgalactopyranose into acetic acid and reacting with hydrobromic acid - acetic acid.

On the other hand, a pyridone derivative (4) used as a starting material in the present invention is
(S)-(-)-3-(1-methyl-2-pyrrolidinyl)-2-pyridone (4a),
(R)-(+)-3-(1-methyl-2-pyrrolidinyl)-2-pyridone (4b),
(S)-(-)-5-(1-methyl-2-pyrrolidinyl)-2-pyridone (4c), and
(R)-(+)-5-(1-methyl-2-pyrrolidinyl)-2-pyridone (4d).
(4a),(4b),(4c) and (4d) can be relatively easily obtained by optical resolution of 3-(1-methyl-2-pyrrolidinyl)-2-pyridone (4e), and 5-(1-methyl-2-pyrrolidinyl)-2-pyridone (4f) prepared by a method described in Chem. Ber., 57B, 1163 (1924), using an optically active column as shown in the following reaction scheme. In this way, a raw material to prepare the compound (1) of the present invention can be relatively easily obtained.

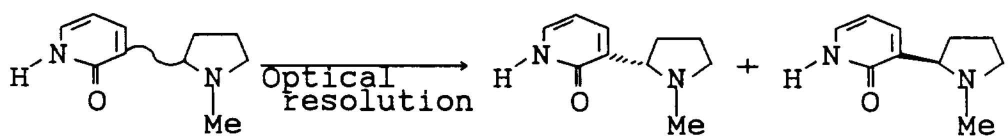


(4e) (4a) (4b)

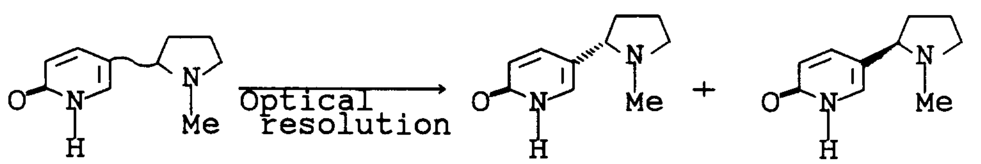


(4f) (4c) (4d)

Hereinbelow, the method of manufacturing the compounds of the present invention is concretely described with reference to methods of manufacturing particularly preferable compounds from (1a) to (1h).

[A] (S)-(-)-3-(1-methyl-2-pyrrolidinyl)-2-pyridyl-β-D-glucopyranoside (1a)

A glycosyl donor,
(2,3,4,6-tetra-O-acetylglucopyranosyl bromide; 3a) and (S)-(-)-3-(1-methyl-2-pyrrolidinyl)-2-pyridone (4a) are dissolved in a solvent such as chloroform and methylene chloride which is used in general glycosylation, and react in the presence of a silver compound such as silver carbonate and silver trifluoromethanesulfonate, thereby obtaining a compound (5a). The obtained product can be purified by a purification means such as column chlomatography.

The obtained product (5a) is dissolved in a solvent such as methanol and treated with a base such as ammonia, sodium methoxide or the like, thereby obtaining the compound (1a) in a high yield. The obtained product can be purified by a purification means such as column chromatography.

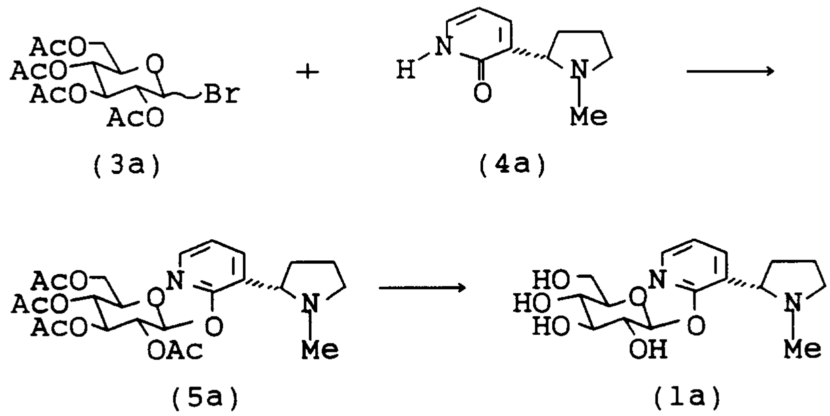

(3a) (4a)

(5a) (1a)

The compound (5a) is characterized in that it has an (S)-(-)-3-(1-methyl-2-pyrrolidinyl)-2-pyridyl group as an aglycone and a 2,3,4,6-tetra-O-acetylglucopyranosyl group as a glycosyl part, both of which are bound with a $\beta$-O-glycoside bond. The compound (5a) is an important intermediate so as to prepare a compound (1a) as well as a nicotine derivative having a useful property by itself.

[B] (R)-(+)-3-(1-methyl-2-pyrrolidinyl)-2-pyridyl-$\beta$-D-glucopyranoside (1b)

As shown in the following reaction scheme, the compound (1b) is manufactured using a glycosyl donor (2,3,4,6-tetra-O-acetylglucopyranosyl bromide; 3a) and (R)-(+)-3-(1-methyl-2-pyrrolidinyl)-2-pyridone (4b), as starting material, and performing a reaction in the same manner as that in the above-described [A].

The compounds (5b) and (1b) can be purified by a purification means such as column chromatography.

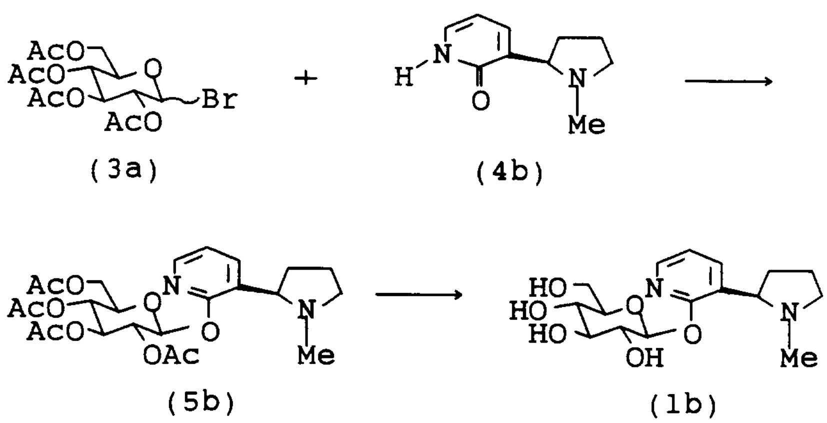

(3a) (4b)

(5b) (1b)

The compound (5b) is characterized in that it has an (R)-(+)-3-(1-methyl-2-pyrrolidinyl)-2-pyridyl group as an aglycone and a 2,3,4,6-tetra-O-acetylglucopyranosyl group as a glycosyl part, both of which are bound with a $\beta$-O-glycoside bond. The compound (5b) is an important intermediate to prepare the compound (1b) as well as a nicotine derivative having a useful property by itself.

[C] (S)-(-)-3-(1-methyl-2-pyrrolidinyl)-2-pyridyl-$\beta$-D-galactopyranoside (1c)

As shown in the following reaction scheme, the compound (1c) is manufactured using a glycosyl donor (2,3,4,6-tetra-O-acetylgalactopyranosyl bromide; 3b) and (S)-(-)-3-(1-methyl-2-pyrrolidinyl)-2-pyridone (4a) as starting material and performing a reaction in the same manner as that in the above-described [A].

The compounds (5c) and (1c) can be purified by a purification means such as column chromatography.

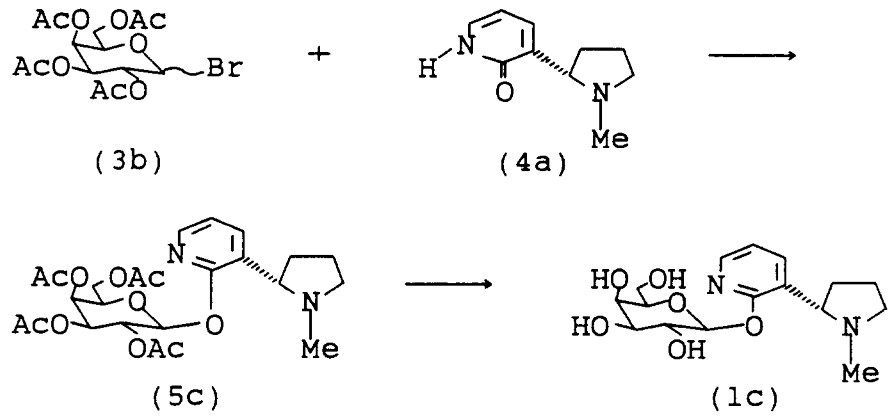

(3b) (4a) (5c) (1c)

The compound (5c) is characterized in that it has an (S)-(-)-3-(1-methyl-2-pyrrolidinyl)-2-pyridyl group as an aglycone and a 2,3,4,6-tetra-O-acetylgalactopyranosyl group as a glycosyl part, both of which are bound with a $\beta$-O-glycoside bond. The compound (5c) is an important intermediate so as to prepare the compound (1c) as well as, a nicotine derivative having a useful property by itself.

[D] (R)-(+)-3-(1-methyl-2-pyrrolidinyl)-2-pyridyl-$\beta$-D-galactopyranoside (1d)

As shown in the following reaction scheme, the compound (1d) is manufactured using a glycosyl donor (2,3,4,6-tetra-O-acetylgalactopyranosyl bromide; 3b) and (R)-(+)-3-(1-methyl-2-pyrrolidinyl)-2-pyridone (4b), as starting material and performing a reaction in the same manner as that in the above-described [A].

The compounds (5d) and (1d) can be purified by a purification means such as column chromatography.

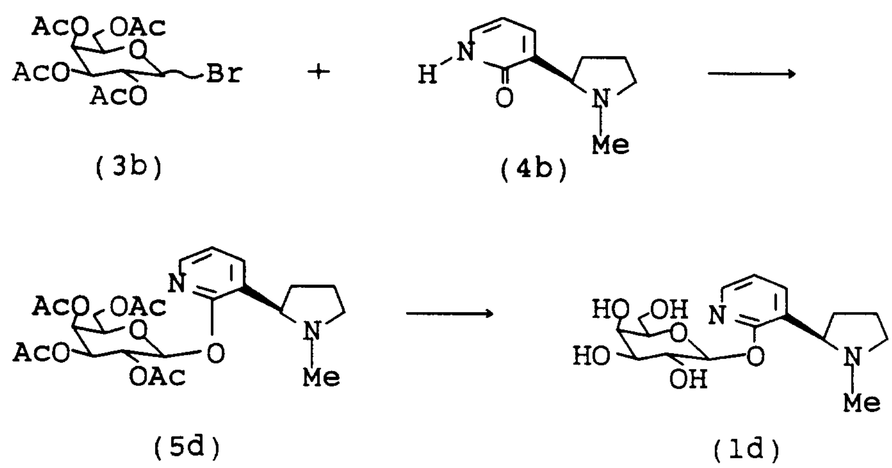

(3b) (4b) (5d) (1d)

The compound (5d) is characterized in that it has an (R)-(+)-3-(1-methyl-2-pyrrolidinyl)-2-pyridyl group as an aglycone and a 2,3,4,6-tetra-O-acetylgalactopyranosyl group as a glycosyl part, both of which are bound with a $\beta$-O-glycoside bond. The compound (5d) is an important intermediate so as to prepare a compound (1d) as well as a nicotine derivative having a useful property by itself.

[E] (S)-(-)-5-(1-methyl-2-pyrrolidinyl)-2-pyridyl-$\beta$-D-glucopyranoside (1e)

As shown in the following reaction scheme, the compound (1e) is manufactured using a glycosyl donor (2,3,4,6-tetra-O-acetylglucopyranosyl bromide; 3a) and (S)-(-)-5-(1-methyl-2-pyrrolidinyl)-2-pyridone (4c) as starting material and performing a reaction in the same manner as in the above-described [A].

The compounds (5e) and (1e) can be purified by a purification means such as column chromatography.

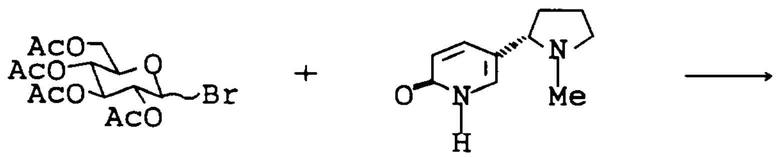

(3a) (4c)

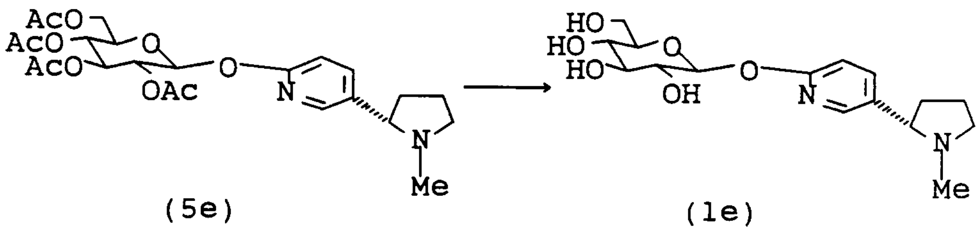

(5e) (1e)

The compound (5e) is characterized in that it has an (S)-(-)-5-(1-methyl-2-pyrrolidinyl)-2-pyridyl group as an aglycone and a 2,3,4,6-tetra-O-acetylglucopyranosyl group as a glycosyl part, both of which are bound with a β-O-glycoside bond. The compound (5e) is an important intermediate so as to prepare a compound (1e) as well as a nicotine derivative having a useful property by itself.

[F] (R)-(+)-5-(1-methyl-2-pyrrolidinyl)-2-pyridyl-β-D-glucopyranoside (1f)

As shown in the following reaction scheme, the compound (1f) is manufactured using a glycosyl donor (2,3,4,6-tetra-O-acetylglucopyranosyl bromide; 3a) and (R)-(+)-5-(1-methyl-2-pyrrolidinyl)-2-pyridone (4d) as starting material and performing a reaction in the same manner as in the above-described [A].

The compounds (5f) and (1f) can be purified by a purification means such as column chromatography.

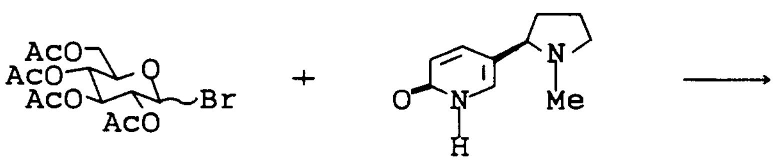

(3a) (4d)

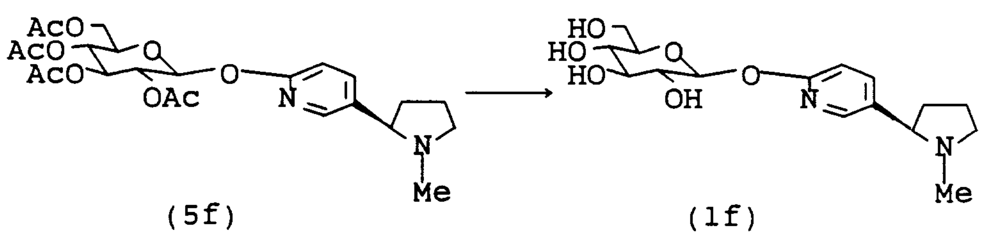

(5f) (1f)

The compound (5f) is characterized in that it has an (R)-(+)-5-(1-methyl-2-pyrrolidinyl)-2-pyridyl group as an aglycone and a 2,3,4,6-tetra-O-acetylglucopyranosyl group as a glycosyl part, both of which are bound with a β-O-glycoside bond. The compound (5f) is an important intermediate so as to prepare a compound (1f) as well as a nicotine derivative having a useful property by itself.

[G] (S)-(-)-5-(1-methyl-2-pyrrolidinyl)-2-pyridyl-β-D-galactopyranoside (1g)

As shown in the following reaction scheme, the compound (1g) is manufactured using a glycosyl donor (2,3,4,6-tetra-O-acetylgalactopyranosyl bromide; 3b) and (S)-(-)-5-(1-methyl-2-pyrrolidinyl)-2-pyridone (4c),

as starting material and performing a reaction in the same manner as in the above-described [A].

The compounds (5g) and (1g) can be purified by a purification means such as column chromatography.

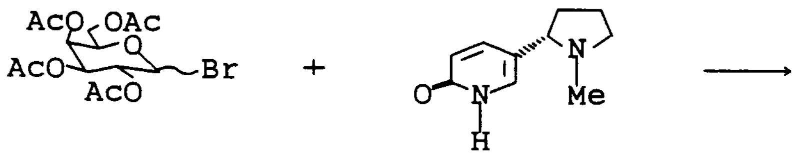

(3b) (4c)

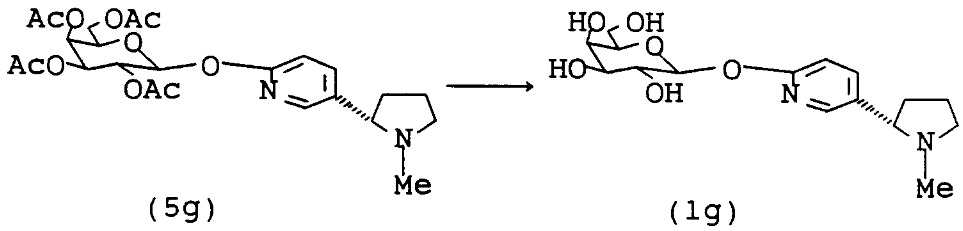

(5g) (1g)

The compound (5g) is characterized in that it has an (S)-(-)-5-(1-methyl-2-pyrrolidinyl)-2-pyridyl group as an aglycone and a 2,3,4,6-tetra-O-acetylgalactopyranosyl group as a glycosyl part, both of which are bound with a β-O-glycoside bond. The compound (5g) is an important intermediate so as to prepare a compound (1g) as well as a nicotine derivative having a useful property by itself.

[H] (R)-(+)-3-(1-methyl-2-pyrrolidinyl)-2-pyridyl-β-D-galactopyranoside (1h)

As shown in the following reaction scheme, the compound (1h) is manufactured using a glycosyl donor (2,3,4,6-tetra-O-acetylgalactopyranosyl bromide; 3b) and (R)-(+)-5-(1-methyl-2-pyrrolidinyl)-2-pyridone (4d), as starting material and performing a reaction in the same manner as in the above-described [A].

The compounds (5h) and (1h) can be purified by a purification means such as column chromatography.

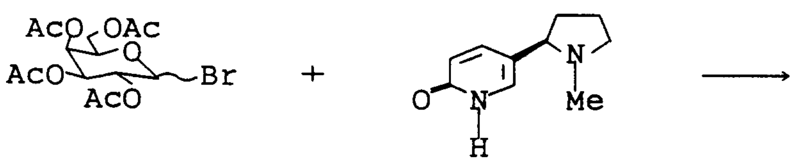

(3b) (4d)

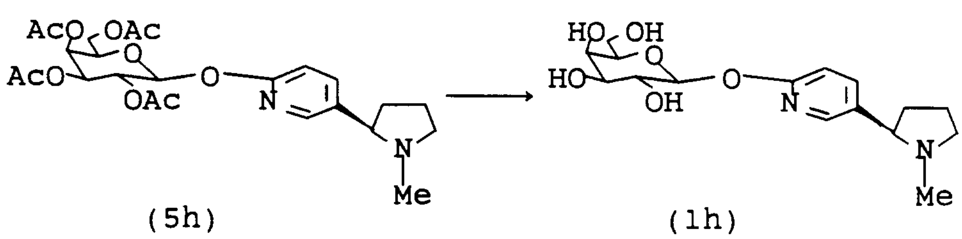

(5h) (1h)

The compound (5h) is characterized in that it has an (R)-(+)-5-(1-methyl-2-pyrrolidinyl)-2-pyridyl group as an aglycone and a 2,3,4,6-tetra-O-acetylgalactopyranosyl group as a glycosyl part, both of which are bound with a β-O-glycoside bond. The compound (5h) is an important intermediate so as to prepare a

compound (1h) as well as a nicotine derivative having a useful property by itself.

The compound (1) of the present invention is expected to have a variety of physiological properties such as an autonomic nervous regulatory activity, a circulatory-system regulatory activity such as vasopressor activity, or a digestive organ regulatory activity such as gastrointestinal activation. However, as the most practical usage, there may be mentioned a flavor and taste improving agent. The evaluation of the usage as a flavor and taste improving agent with respect to the compound (1) of the present invention can be, for example, performed in the following tests 1) and 2).

1) Effect on flavor, taste, and stimulation

An ethanol solution of the compound (1) of the present invention is prepared and spray-added to a shredded tobacco leaf filling. This tobacco leaf filling is rolled up and compared with an unsprayed tobacco filling used as a control in terms of flavor, taste, and stimulation by a discrimination method.

2) Effect on flavor and taste

Two types of the compound (1) are mixed and its ethanol solution is prepared. The ethanol solution is spray-added to a shredded tobacco leaf filling. This tobacco leaf filing is rolled up and compared with an unsprayed tobacco leaf filling used as a control in terms of flavor and taste at the time of smoking by a discrimination method.

When usefulness on improving flavor and taste with respect to the preferable compounds (1a) to (1h) of the present invention was tested, it was demonstrated that they are substances excellent as an agent improving for flavor and taste.

[Brief Description of drawings]

FIG. 1 is a reference view of the X-ray crystal structure analysis data of a compound (5b); and
FIG. 2 is a reference view of the X-ray crystal structure analysis data of a compound (5e).

[Best Mode of Carrying out the Invention]

Hereinbelow, the present invention will be described in detail with reference to Examples, which should not construed as limiting the scope of the present invention.

Synthesis of Starting Material

[1] 2,3,4,6-tetra-O-acetylglucopyranosyl bromide (3a)(Chem. Ber., 57B, 1163 (1924)), 3-(1-methyl-2-pyrrolidinyl)-2-pyridone (4e), and 5-(1-methyl-2-pyrrolidinyl)-2-pyridone (4f)(Nature, 165, 369 (1950)) were synthesized according to the conventional method. Further, 2,3,4,6-tetra-O-acetylgalactopyranosyl bromide (3b) was prepared by dissolving commercially available 1,2,3,4,6-penta-O-acetylgalactopyranoside in acetic acid and reacting with hydrobromic acid - acetic acid.

[2] Preparation of (S)-(-)-3-(1-methyl-2-pyrrolidinyl)-2-pyridone (4a), and (R)-(+)-3-(1-methyl-2-pyrrolidinyl) -2-pyridone (4b)

(S)-(-)-3-(1-methyl-2-pyrrolidinyl)-2-pyridone (4a) and (R)-(+)-3-(1-methyl-2-pyrrolidinyl)-2-pyridone (4b) were obtained by resolving 3-(1-methyl-2-pyrrolidinyl)-2-pyridone (4e) which has been obtained by the above-described conventional method, by use of an optically active column.

Separation condition

| | |
|---|---|
| Column: | CHIRALPAK AS (Daisel Chemical Industries, LTD) |
| Solvent: | hexan/2-propanol = 9/1 |
| Retention time: | (S)-(-)-3-(1-methyl-2-pyrrolidinyl)-2-pyridone (4a); 19 minutes;<br>(R)-(+)-3-(1-methyl-2-pyrrolidinyl)-2-pyridone (4b); 14 minutes |

Physical properties

(S)-(-)-3-(1-methyl-2-pyrrolidinyl)-2-pyridone (4a)

$[\alpha]_D$ -197° (c 0.93, methanol)

Melting point: 95.0 - 96.0 °C
(R)-(+)-3-(1-methyl-2-pyrrolidinyl)-2-pyridone (4b)
$[\alpha]_D$ +207° (c 0.89, methanol)
Melting point: 93.0 - 93.5 °C

The values of physical properties of the compounds (4a) and (4b) are identical as shown in the following.

| | |
|---|---|
| IR: | $\nu$ ($cm^{-1}$); 3136, 2946, 2778, 1647, 1618, 1562, 1477, 1164, 1052, 766 |
| $^1$H-NMR: | $\delta$ ($CDCl_3$; ppm from TMS) |
| | 1.53 - 1.57 (1H, m), |
| | 1.80 - 1.87 (2H, m), |
| | 2.26 (3H, s), |
| | 2.40 (2H, m), |
| | 3.23 (1H, m), |
| | 3.49 (1H, t, J = 8.3 Hz), |
| | 6.34 (1H, dd, J = 6.4, 7.0 Hz), |
| | 7.36 (1H, dd, J = 2.0, 6.4 Hz), |
| | 7.65 (1H, dd, J = 2.0, 7.0 Hz) |
| $^{13}$C-NMR: | $\delta$ ($CDCl_3$; ppm from TMS); |
| | 22.8 ($CH_2$), 33.1 ($CH_2$), 40.8 ($CH_3$), 56.1 ($CH_2$), 64.1 (CH), 107.2 (CH), 132.7 (CH), 134.0 (C), 136.8 (CH), 165.0 (C) |
| MS (%): | 178 ($M^+$) (23), 177 (11), 163 (64), 149 (29), 122 (11), 120 (11), 108 (12), 84 (100), 57 (13) |

[3] (S)-(-)-5-(1-methyl-2-pyrrolidinyl)-2-pyridone (4c) and (R)-(+)-5-(1-methyl-2-pyrrolidinyl)-2-pyridone (4d)

(S)-(-)-5-(1-methyl-2-pyrrolidinyl)-2-pyridone (4c) and (R)-(+)-5-(1-methyl-2-pyrrolidinyl)-2-pyridone (4d) were obtained by resolving 5-(1-methyl-2-pyrrolidinyl)-2-pyridone (4f) which has been obtained by the above-described conventional method, by use of an optically active column.

Separation condition

| | |
|---|---|
| Column: | CHIRALPAK AS (Daisel Chemical Industries, LTD) |
| Solvent: | hexan/ethanol = 9/1 |
| Retention time: | (S)-(-)-5-(1-methyl-2-pyrrolidinyl)-2-pyridone (4c); 40 minutes; |
| | (R)-(+)-5-(1-methyl-2-pyrrolidinyl)-2-pyridone (4d); 50 minutes |

Physical properties

(S)-(-)-5-(1-methyl-2-pyrrolidinyl)-2-pyridone (4c)
$[\alpha]_D$ -117° (c 0.87, methanol)
Melting point: 123.0 - 124.0 °C
(R)-(+)-5-(1-methyl-2-pyrrolidinyl)-2-pyridone (4d)
$[\alpha]_D$ +111° (c 0.71, methanol)
Melting point: 123.5 - 124.5 °C

The values of physical properties of the compounds (4c) and (4d) are identical as shown in the following.

| | |
|---|---|
| IR: | $\nu$ ($cm^{-1}$); 3126, 2948, 2782, 1661, 1624, 1549, 1462, 1307, 1218, 1044, 837, 600 |
| $^1$H-NMR: | $\delta$ ($CDCl_3$; ppm from TMS) |
| | 1.70 - 1.85 (2H, m), |
| | 1.85 - 1.95 (1H, m), |
| | 2.00 - 2.10 (1H, s), |
| | 2.15 (3H, s), |
| | 2.25 (1H, q, J = 7.6 Hz), |
| | 2.84 (1H, t, J = 8.2 Hz), |
| | 3.19 (1H, t, J = 7.6 Hz), |
| | 6.60 (1H, d, J = 9.4 Hz), |
| | 7.30 (1H, d, J = 2.5 Hz), |

7.57 (1H, dd, J = 2.5, 9.4 Hz)

$^{13}$C-NMR: δ ($CDC\ell_3$; ppm from TMS); 22.5 ($CH_2$), 33.9 ($CH_2$), 40.2 ($CH_3$), 56.7 ($CH_2$), 67.7 (CH), 120.5 (CH), 121.9 (C), 132.8 (CH), 141.5 (CH), 165.6 (C)

MS (%): 179 (55), 178 ($M^+$) (100), 177 (85), 150 (24), 149 (90), 135 (22), 122 (14), 121 (16), 84 (61)

Example 1

Synthesis of (S)-(-)-3-(1-methyl-2-pyrrolidinyl) -2-pyridyl-β-D-glucopyranoside (1a)

1. (S)-(-)-3-(1-methyl-2-pyrrolidinyl)-2-pyridyl 2,3,4,6-tetra-O-acetyl β-D-glucopyranoside (5a)

2.0g of (S)-(-)-3-(1-methyl-2-pyrrolidinyl)-2-pyridone (4a) and 5.6g of 2,3,4,6-tetra-O-acetylglucopyranosyl bromide (3a) were dissolved in 40 mℓ of dichloromethane. To the solution, 1.8g of silver carbonate was added, followed by shielding the light with aluminum sheet, the mixture was stirred at room temperature for a day and a night. Solid material was filtered off and the filtrate was concentrated under reduced pressure. The residue was purified by means of silica gel column chromatography (ethyl acetate), obtaining 5.0g of (S)-(-)-3-(1-methyl-2-pyrrolidinyl)-2-pyridyl 2,3,4,6-tetra-O-acetyl-β-D-glucopyranoside (5a) in white solid form. This was recrystallized from ethyl acetate - hexan to give compound (5a) in high purity (4.9g; 88%)

Physical properties

Melting point: 149.0 - 151.0 °C
$[\alpha]_D$ - 68.1 ° (c 0.75, methanol)
IR: ν ($cm^{-1}$); 2948, 2784, 1756, 1589, 1437, 1367, 1230
$^1$H-NMR: δ ($CDC\ell_3$; ppm from TMS)
1.5 - 1.6 (1H, m),
1.8 - 1.9 (2H, m),
1.98 - (3H, s),
2.04 (3H, s),
2.05 (6H, s),
2.15 (3H, s),
2.32 (2H, m),
3.20 (1H, ddd, J = 2.4, 6.9, 9.2 Hz),
3.25 (1H, t, J = 8.3 Hz),
3.96 (1H, ddd, J = 2.3, 4.3, 9.9 Hz),
4.14 (1H, dd, J = 2.3, 12.4 Hz),
4.31 (1H, dd, J = 4.3, 12.4 Hz),
5.23 (1H, m),
5.36 (2H, m),
6.14 (1H, d, J = 8.0 Hz),
7.01 (1H, dd, J = 4.9, 7.4 Hz),
7.83 (1H, dd, J = 1.9, 7.4 Hz),
8.02 (1H, dd, J = 1.9, 4.9 Hz)
$^{13}$C-NMR: δ ($CDC\ell_3$; ppm from TMS); 20.8 ($CH_3$), 23.0 ($CH_2$) 33.7 ($CH_2$), 40.8 ($CH_3$), 57.1 ($CH_2$), 61.9 ($CH_2$), 63.1 (CH), 68.5 (CH), 71.0 (CH), 72.2 (CH), 73.3 (CH), 93.8 (CH), 119.5 (CH), 127.0 (C), 136.9 (CH), 144.7 (CH), 159.4 (C), 169.4 (C), 169.7 (C), 170.4 (C), 170.8 (C)
MS (%): 509 ($M^{+1}$) (19), 508 (17), 389 (22), 179 (18), 178 (19), 177 (100), 169 (25), 163 (20), 109 (20), 84 (42)

In the above physical properties, since disappearance of carbonyl absorption (in the vicinity of 1647 $cm^{-1}$) of the compound (4a) in IR, a characteristic signal of β-O-glycoside (δ 6.14 (1H, d, J = 8.0 Hz) in $^1$H - NMR, and signals (1.98 (3H, s), 2.04 (3H, s) and 2.05 (6H, s)) of acetyl groups are observed, the structure of the compound (5a) can be confirmed.

2. (S)-(-)-3-(1-methyl-2-pyrrolidinyl)-2-pyridyl β-D-glucopyranoside (1a)

4.9g of (S)-(-)-3-(1-methyl-2-pyrrolidinyl)-2-pyridyl 2,3,4,6-tetra-O-acetyl-β-D-glucopyranoside (5a) was dissolved in 20 mℓ of methanol. To this solution, 10 mℓ of methanol saturated with ammonia was added

and the mixture was stirred at room temperature for 2 hours. The reaction mixture was concentrated under reduced pressure, thereby obtaining 3.1g of (S)-(-)-3-(1-methyl-2-pyrrolidinyl)-2-pyridyl $\beta$-D-glucopyranoside (1a) in white solid form.

Physical properties

$[\alpha]_D$ -82.7° (c 0.64, methanol)
UV: $\lambda_{max}$ 219 nm ($\epsilon$ = 4,700), 272 nm ($\epsilon$ = 4,100)
IR: $\nu$ ($cm^{-1}$); 3356, 2924, 1591, 1437, 1357, 1243, 1052
$^1$H-NMR: $\delta$ ($CDCl_3$; ppm from TMS)
1.95 - 2.05 (3H, m)
2.22 (3H, s),
2.32 (1H, m),
2.34 (1H, q, J = 9.1 Hz),
3.28 (1H, m),
3.45 (1H, t, J = 9.0 Hz),
3.5 - 3.6 (4H, m),
3.76 (1H, dd, J = 5.1, 11.9 Hz),
3.93 (1H, dd, J = 1.8, 11.9 Hz),
5.66 (1H, d, J = 7.4 Hz),
7.12 (1H, dd, J = 5.0, 7.4 Hz),
7.82 (1H, dd, J = 1.8, 7.4 Hz),
8.14 (1H, dd, J = 1.8, 5.0 Hz)
$^{13}$C - NMR: $\delta$ ($CDCl_3$; ppm from TMS)
23.9 ($CH_2$), 31.2 ($CH_2$), 41.0 ($CH_3$), 58.1 ($CH_2$), 62.6 ($CH_2$), 68.5 (CH), 71.0 (CH), 74.9 (CH), 77.6 (CH), 78.6 (CH), 99.7 (CH), 120.1 (CH), 126.2 (C), 140.4 (CH), 146.8 (CH), 162.2 (C)
MS (%): 341 ($M^{+1}$) (1.2), 340 (1.8), 235 (2), 179 (75), 178 (17), 177 (57), 163 (89), 149 (20), 148 (17), 84 (100)

HRMS (high resolution mass spectrum):
Obs.; 341.1761
cald. for; $C_{12}H_{25}N_2O_6$ ($M^{+1}$) 341.1713

In the above physical properties, since disappearance of carbonyl absorption (in the vicinity of 1647 $cm^{-1}$) of the compound (4a) in IR, and a characteristic signal of $\beta$-O-glycoside ($\delta$ 5.66 (1H, d, J = 7.4 Hz)) in $^1$H - NMR are observed, the structure of the compound (1a) can be confirmed.

Example 2

Synthesis of (R)-(+)-3-(1-methyl-2-pyrrolidinyl)-2-pyridyl $\beta$-D-glucopyranoside (1b)

1. (R)-(+)-3-(1-methyl-2-pyrrolidinyl)-2-pyridyl 2,3,4,6-tetra-O-acetyl-$\beta$-D-glucopyranoside (5b)

Using 5g of (R)-(+)-3-(1-methyl-2-pyrrolidinyl)-2-pyridone (4b) and 5.6g of 2,3,4,6-tetra-O-acetylglucopyranosyl bromide (3a) as starting material, (R)-(+)-3-(1-methyl-2-pyrrolidinyl)-2-pyridyl 2,3,4,6-tetra-O-acetyl-$\beta$-D-glucopyranoside (5b)(4.8g) was obtained in the same manner as in 1 of Example 1.

Physical properties

Melting point: 132.5-134.0°C
$[\alpha]_D$: + 46.4° (c 0.64, methanol)
IR: $\nu$ ($cm^{-1}$); 2948, 2786, 1756, 1589, 1437, 1369, 1232, 1040, 911, 733, 600
$^1$H-NMR: $\delta$ ($CDCl_3$; ppm from TMS)
1.35 - 1.45 (1H, m),
1.75 - 1.85 (2H, m),
1.98 (3H, s),
2.04 (3H, s),
2.05 (3H, s),

2.06 (3H, s),
2.10 - 2.20 (1H, m),
2.23 (3H, s),
2.35 (1H, q, J = 8.8 Hz),
3.19 (1H, m),
3.47 (1H, t, J = 8.2 Hz),
3.96 (1H, m),
4.13 (1H, dd, J = 2.3, 12.4 Hz),
4.32 (1H, dd, J = 4.4, 12.4 Hz),
5.22 (1H, t, J = 9.2 Hz),
5.35 (2H, m),
6.20 (1H, d, J = 7.6 Hz),
7.00 (1H, dd, J = 4.9, 7.4 Hz),
7.84 (1H, dd, J = 1.9, 7.4 Hz),
8.01 (1H, dd, J = 1.9, 4.9 Hz)

$^{13}$C-NMR: δ (CDCℓ$_3$; ppm from TMS); 20.8 ($CH_3$), 22.9 ($CH_2$) 33.1 ($CH_2$), 41.0 ($CH_3$), 57.1 ($CH_2$), 61.8 ($CH_2$), 63.0 (CH), 68.4 (CH), 70.8 (CH), 72.2 (CH), 73.4 (CH), 93.3 (CH), 119.3 (CH), 127.3 (C), 136.3 (CH), 144.3 (CH), 159.1 (C), 169.5 (C), 169.6 (C), 170.4 (C), 170.8 (C)

MS (%): 509 ($M^{+1}$) (26), 508 (14), 389 (17), 179 (20), 178 (19), 177 (100), 169 (26), 163 (24), 109 (27), 84 (45)

In the above physical properties, since disappearance of carbonyl absorption (in the vicinity of 1647 $cm^{-1}$) of the compound (4a) in IR, a characteristic signal of β-O-glycoside (δ 6.20 (1H, d, J = 7.6 Hz)) in $^1$H - NMR, and signals (1.98 (3H, s), 2.04 (3H, s), 2.05 (3H, s) and 2.06 (3H, s)) of acetyl groups are observed, the structure of the compound (5b) can be confirmed. Further, it is also confirmed the structure of the compound (5b) from the results of the X-ray crystal structure analysis.

X-Ray diffraction data of compound (5b)

bond distance

| | | | | | |
|---|---|---|---|---|---|
| $^{1}N - ^{2}C$ | 1.471 | $^{5}C - ^{5}Ha$ | 1.118 | $^{11}C - ^{12}C$ | 1.360 |
| $^{1}N - ^{5}C$ | 1.423 | $^{5}C - ^{5}Hb$ | 1.113 | $^{11}C - ^{11}H$ | 1.007 |
| $^{1}N - ^{6}C$ | 1.480 | $^{6}C - ^{6}Ha$ | 1.106 | $^{12}C - ^{12}H$ | 1.077 |
| $^{2}C - ^{3}C$ | 1.502 | $^{6}C - ^{6}Hb$ | 1.181 | $^{13}C - ^{36}O$ | 1.403 |
| $^{2}C - ^{9}C$ | 1.501 | $^{6}C - ^{6}Hc$ | 1.400 | $^{13}C - ^{18}O$ | 1.423 |
| $^{2}C - ^{2}H$ | 0.982 | $^{7}N - ^{8}C$ | 1.321 | $^{13}C - ^{14}C$ | 1.494 |
| $^{3}C - ^{4}C$ | 1.550 | $^{7}N - ^{12}C$ | 1.334 | $^{13}C - ^{13}H$ | 1.002 |
| $^{3}C - ^{3}Ha$ | 1.136 | $^{8}C - ^{9}C$ | 1.392 | $^{14}C - ^{15}C$ | 1.533 |
| $^{3}C - ^{3}Hb$ | 0.945 | $^{8}C - ^{36}O$ | 1.379 | $^{14}C - ^{24}O$ | 1.435 |
| $^{4}C - ^{5}C$ | 1.447 | $^{9}C - ^{10}C$ | 1.368 | $^{14}C - ^{14}H$ | 0.955 |
| $^{4}C - ^{4}Ha$ | 0.982 | $^{10}C - ^{11}C$ | 1.365 | $^{15}C - ^{16}C$ | 1.521 |
| $^{4}C - ^{4}Hb$ | 0.768 | $^{10}C - ^{10}H$ | 0.781 | $^{15}C - ^{28}O$ | 1.441 |
| $^{15}C - ^{15}H$ | 0.992 | $^{21}C - ^{23}O$ | 1.163 | $^{29}C - ^{31}O$ | 1.187 |
| $^{16}C - ^{17}C$ | 1.533 | $^{22}C - ^{22}Ha$ | 0.853 | $^{30}C - ^{30}Ha$ | 0.844 |
| $^{16}C - ^{32}O$ | 1.440 | $^{22}C - ^{22}Hb$ | 1.186 | $^{30}C - ^{30}Hb$ | 0.932 |
| $^{16}C - ^{16}H$ | 0.965 | $^{22}C - ^{22}Hc$ | 0.569 | $^{30}C - ^{30}Hc$ | 0.752 |

| | | | | | |
|---|---|---|---|---|---|
| $^{17}C$ - $^{18}O$ | 1.434 | $^{24}O$ - $^{25}C$ | 1.335 | $^{32}O$ - $^{33}C$ | 1.357 |
| $^{17}C$ - $^{19}C$ | 1.499 | $^{25}C$ - $^{26}C$ | 1.481 | $^{33}C$ - $^{34}C$ | 1.486 |
| $^{17}C$ - $^{17}H$ | 0.990 | $^{25}C$ - $^{27}O$ | 1.164 | $^{33}C$ - $^{35}O$ | 1.190 |
| $^{19}C$ - $^{20}O$ | 1.434 | $^{26}C$ - $^{26}Ha$ | 1.086 | $^{34}C$ - $^{34}Ha$ | 1.002 |
| $^{19}C$ - $^{19}Ha$ | 0.985 | $^{26}C$ - $^{26}Hb$ | 0.783 | $^{34}C$ - $^{34}Hb$ | 0.991 |
| $^{19}C$ - $^{19}Hb$ | 0.916 | $^{26}C$ - $^{26}Hc$ | 0.921 | $^{34}C$ - $^{34}Hc$ | 1.016 |
| $^{20}O$ - $^{21}C$ | 1.349 | $^{28}O$ - $^{29}C$ | 1.352 | | |
| $^{21}C$ - $^{22}C$ | 1.495 | $^{29}C$ - $^{30}C$ | 1.483 | | |

bond angle

| | | | | | |
|---|---|---|---|---|---|
| $^{2}C-^{1}N-^{5}C$ | 105.65 | $^{5}C-^{4}C-^{4}Hb$ | 111.45 | $^{8}C-^{9}C-^{2}C$ | 121.11 |
| $^{2}C-^{1}N-^{6}C$ | 114.39 | $^{4}Ha-^{4}C-^{4}Hb$ | 103.50 | $^{2}C-^{9}C-^{10}C$ | 123.33 |
| $^{5}C-^{1}N-^{6}C$ | 113.87 | $^{4}C-^{5}C-^{1}N$ | 104.72 | $^{9}C-^{10}C-^{11}C$ | 120.81 |
| $^{1}N-^{2}C-^{3}C$ | 104.43 | $^{4}C-^{5}C-^{5}Ha$ | 111.32 | $^{9}C-^{10}C-^{10}H$ | 127.82 |
| $^{1}N-^{2}C-^{9}C$ | 111.04 | $^{4}C-^{5}C-^{5}Hb$ | 98.85 | $^{11}C-^{10}C-^{10}H$ | 111.01 |
| $^{1}N-^{2}C-^{2}H$ | 106.78 | $^{1}N-^{5}C-^{5}Ha$ | 115.25 | $^{10}C-^{11}C-^{12}C$ | 118.88 |
| $^{3}C-^{2}C-^{9}C$ | 114.40 | $^{1}N-^{5}C-^{5}Hb$ | 115.33 | $^{10}C-^{11}C-^{11}H$ | 132.57 |
| $^{3}C-^{2}C-^{2}H$ | 111.32 | $^{5}Ha-^{5}C-^{5}Hb$ | 109.98 | $^{12}C-^{11}C-^{11}H$ | 108.51 |
| $^{9}C-^{2}C-^{2}H$ | 108.57 | $^{1}N-^{6}C-^{6}Ha$ | 118.46 | $^{11}C-^{12}C-^{7}N$ | 122.86 |
| $^{2}C-^{3}C-^{4}C$ | 101.59 | $^{1}N-^{6}C-^{6}Hb$ | 81.36 | $^{11}C-^{12}C-^{12}H$ | 108.57 |
| $^{2}C-^{3}C-^{3}Ha$ | 122.02 | $^{1}N-^{6}C-^{6}Hc$ | 119.59 | $^{7}N-^{12}C-^{12}H$ | 125.64 |
| $^{2}C-^{3}C-^{3}Hb$ | 113.84 | $^{6}Ha-^{6}C-^{6}Hb$ | 124.47 | $^{8}C-^{36}O-^{13}C$ | 118.10 |
| $^{4}C-^{3}C-^{3}Ha$ | 113.50 | $^{6}Ha-^{6}C-^{6}Hc$ | 102.19 | $^{14}C-^{13}C-^{18}O$ | 107.97 |
| $^{4}C-^{3}C-^{3}Hb$ | 107.68 | $^{6}Hb-^{6}C-^{6}Hc$ | 111.26 | $^{14}C-^{13}C-^{36}O$ | 106.96 |
| $^{3}Ha-^{3}C-^{3}Hb$ | 97.97 | $^{8}C-^{7}N-^{12}C$ | 116.84 | $^{14}C-^{13}C-^{13}H$ | 110.14 |
| $^{3}C-^{4}C-^{5}C$ | 108.16 | $^{7}N-^{8}C-^{9}C$ | 125.03 | $^{18}O-^{13}C-^{36}O$ | 107.02 |
| $^{3}C-^{4}C-^{4}Ha$ | 112.52 | $^{7}N-^{8}C-^{36}O$ | 118.62 | $^{18}O-^{13}C-^{13}H$ | 109.10 |

| | | | | | |
|---|---|---|---|---|---|
| $^{3}C-^{4}C-^{4}Hb$ | 116.66 | $^{9}C-^{8}C-^{36}O$ | 116.34 | $^{36}O-^{13}C-^{13}H$ | 115.37 |
| $^{5}C-^{4}C-^{4}Ha$ | 103.79 | $^{8}C-^{9}C-^{10}C$ | 115.54 | $^{13}C-^{14}C-^{15}C$ | 109.86 |
| $^{13}C-^{14}C-^{24}O$ | 109.21 | $^{22}C-^{21}C-^{23}O$ | 126.86 | $^{33}C-^{34}C-^{34}Hc$ | 103.30 |
| $^{13}C-^{14}C-^{14}H$ | 109.89 | $^{21}C-^{22}C-^{22}Ha$ | 106.81 | $^{34}Ha-^{34}C-^{34}Hb$ | 102.99 |
| $^{15}C-^{14}C-^{24}O$ | 107.85 | $^{21}C-^{22}C-^{22}Hb$ | 102.81 | $^{34}Ha-^{34}C-^{34}Hc$ | 105.54 |
| $^{15}C-^{14}C-^{14}H$ | 113.16 | $^{21}C-^{22}C-^{22}Hc$ | 98.33 | $^{34}Hb-^{34}C-^{34}Hc$ | 115.07 |
| $^{24}O-^{14}C-^{14}H$ | 106.73 | $^{22}Ha-^{22}C-^{22}Hb$ | 131.04 | | |
| $^{14}C-^{15}C-^{16}C$ | 109.64 | $^{22}Ha-^{22}C-^{22}Hc$ | 98.38 | | |
| $^{14}C-^{15}C-^{28}O$ | 108.62 | $^{22}Hb-^{22}C-^{22}Hc$ | 114.91 | | |
| $^{14}C-^{15}C-^{15}H$ | 111.77 | $^{14}C-^{24}O-^{25}C$ | 117.80 | | |
| $^{16}C-^{15}C-^{28}O$ | 107.27 | $^{24}O-^{25}C-^{26}C$ | 112.79 | | |
| $^{16}C-^{15}C-^{15}H$ | 110.31 | $^{24}O-^{25}C-^{27}O$ | 121.96 | | |
| $^{28}O-^{15}C-^{15}H$ | 109.10 | $^{26}C-^{25}C-^{27}O$ | 125.14 | | |
| $^{15}C-^{16}C-^{17}C$ | 110.90 | $^{25}C-^{26}C-^{26}Ha$ | 107.10 | | |
| $^{15}C-^{16}C-^{32}O$ | 107.96 | $^{25}C-^{26}C-^{26}Hb$ | 114.10 | | |
| $^{15}C-^{16}C-^{16}H$ | 110.46 | $^{25}C-^{26}C-^{26}Hc$ | 107.77 | | |
| $^{17}C-^{16}C-^{32}O$ | 107.77 | $^{26}Ha-^{26}C-^{26}Hb$ | 134.88 | | |
| $^{17}C-^{16}C-^{16}H$ | 107.35 | $^{26}Ha-^{26}C-^{26}Hc$ | 105.44 | | |

| | | | |
|---|---|---|---|
| $^{32}O-^{16}C-^{16}H$ | 112.40 | $^{26}Hb-^{26}C-^{26}Hc$ | 79.16 |
| $^{16}C-^{17}C-^{19}C$ | 112.14 | $^{15}C-^{28}O-^{29}C$ | 119.22 |
| $^{16}C-^{17}C-^{18}O$ | 108.39 | $^{28}O-^{29}C-^{30}C$ | 110.56 |
| $^{16}C-^{17}C-^{17}H$ | 104.79 | $^{28}O-^{29}C-^{31}O$ | 123.84 |
| $^{19}C-^{17}C-^{18}O$ | 107.69 | $^{30}C-^{29}C-^{31}O$ | 125.54 |
| $^{19}C-^{17}C-^{17}H$ | 110.51 | $^{29}C-^{30}C-^{30}Ha$ | 120.02 |
| $^{18}O-^{17}C-^{17}H$ | 113.37 | $^{29}C-^{30}C-^{30}Hb$ | 107.27 |
| $^{17}C-^{18}O-^{13}C$ | 111.20 | $^{29}C-^{30}C-^{30}Hc$ | 107.90 |
| $^{17}C-^{19}C-^{20}O$ | 108.27 | $^{30}Ha-^{30}C-^{30}Hb$ | 89.53 |
| $^{17}C-^{19}C-^{19}Ha$ | 103.96 | $^{30}Ha-^{30}C-^{30}Hc$ | 111.80 |
| $^{17}C-^{19}C-^{19}Hb$ | 106.47 | $^{30}Hb-^{30}C-^{30}Hc$ | 120.04 |
| $^{20}O-^{19}C-^{19}Ha$ | 112.30 | $^{16}C-^{32}O-^{33}C$ | 117.35 |
| $^{20}O-^{19}C-^{19}Hb$ | 114.80 | $^{32}O-^{33}C-^{34}C$ | 110.98 |
| $^{19}Ha-^{19}C-^{19}Hb$ | 110.26 | $^{32}O-^{33}C-^{35}O$ | 123.14 |
| $^{19}C-^{20}O-^{21}C$ | 117.98 | $^{34}C-^{33}C-^{35}O$ | 125.87 |
| $^{20}O-^{21}C-^{22}C$ | 109.49 | $^{33}C-^{34}C-^{34}Ha$ | 110.93 |
| $^{20}O-^{21}C-^{23}O$ | 123.65 | $^{33}C-^{34}C-^{34}Hb$ | 118.49 |

Cartesian co-ordinaates

| | x | y | z |
|---|---|---|---|
| $^{1}N$ | -4.22058 | -8.33454 | -1.70886 |
| $^{2}C$ | -3.31343 | -7.20563 | -1.96711 |
| 2H | -3.60017 | -6.47150 | -1.38056 |
| $^{3}C$ | -1.95691 | -7.71856 | -1.57675 |
| $^{3}Ha$ | -1.13262 | -7.02626 | -1.21478 |
| $^{3}Hb$ | -1.48396 | -8.12579 | -2.28619 |
| $^{4}C$ | -5.65585 | -7.97544 | -1.69981 |
| $^{4}Ha$ | -2.06476 | -8.59573 | 0.35995 |
| $^{4}Hb$ | -2.00239 | -9.51595 | -0.66734 |
| $^{5}C$ | -3.76654 | -8.92185 | -0.49532 |
| $^{5}Ha$ | -4.09759 | -9.97525 | -0.32242 |
| $^{5}Hb$ | -3.93822 | -8.31384 | 0.42148 |
| $^{6}C$ | -5.65585 | -7.97544 | -1.69981 |
| $^{6}Ha$ | -6.40202 | -8.78819 | -1.62272 |
| $^{6}Hb$ | -5.29985 | -7.30632 | -0.79356 |
| $^{6}Hc$ | -6.17900 | -7.19102 | -2.73536 |
| $^{7}N$ | -2.92873 | -5.04631 | -5.02542 |
| $^{8}C$ | -2.86954 | -5.53211 | -3.79869 |
| $^{3}C$ | -3.41628 | -6.74670 | -3.39268 |
| $^{10}C$ | -4.00703 | -7.48709 | -4.38006 |
| $^{10}H$ | -4.41207 | -8.15052 | -4.30385 |
| $^{11}C$ | -4.06632 | -7.02193 | -5.66225 |
| $^{11}H$ | -4.41635 | -7.42324 | -6.51708 |
| $^{12}C$ | -3.53161 | -5.80282 | -5.94450 |
| $^{12}H$ | -3.95444 | -5.44880 | -6.86947 |
| $^{36}O$ | -2.20968 | -4.80445 | -2.83126 |
| $^{13}C$ | -1.72340 | -3.53500 | -3.17644 |
| $^{13}H$ | -2.31435 | -2.99915 | -3.78234 |
| $^{14}C$ | -1.49013 | -2.79547 | -1.89934 |
| $^{14}H$ | -0.96157 | -3.32708 | -1.30786 |
| $^{15}C$ | -0.86930 | -1.42591 | -2.19653 |
| $^{15}H$ | -1.51246 | -0.82048 | -2.64720 |
| $^{16}C$ | 0.37647 | -1.60377 | -3.05154 |
| $^{15}H$ | 1.07468 | -2.03669 | -2.54453 |

| | | | |
|---|---|---|---|
| 17C | 0.08200 | -2.48640 | -4.27036 |
| 17H | -0.55250 | -1.95846 | -4.81745 |
| 18O | -0.46321 | -3.72926 | -3.80774 |
| 19C | 1.32063 | -2.79661 | -5.05574 |
| 19Ha | 1.00493 | -3.39728 | -5.77028 |
| 19Hb | 1.60758 | -2.00338 | -5.41200 |
| 20O | 2.25890 | -3.43765 | -4.18105 |
| 21C | 3.56827 | -3.36834 | -4.49740 |
| 22C | 4.37048 | -4.08884 | -3.46159 |
| 22Ha | 4.26074 | -4.92279 | -3.60082 |
| 22Hb | 4.19566 | -3.43189 | -2.48942 |
| 22Hc | 4.88364 | -4.03171 | -3.70033 |
| 23O | 3.97633 | -2.81352 | -5.43486 |
| 24O | -2.74804 | -2.57547 | -1.24440 |
| 25C | -2.86067 | -2.95438 | 0.03059 |
| 26C | -4.19138 | -2.64842 | 0.60475 |
| 26Ha | -4.52472 | -1.71580 | 0.15910 |
| 26Hb | -4.34747 | -3.02933 | 1.27086 |
| 26Hc | -4.78189 | -3.28925 | 0.30369 |
| 27O | -1.96606 | -3.41190 | 0.61890 |
| 28O | -0.45284 | -0.82974 | -0.95257 |
| 29C | -1.24331 | 0.09809 | -0.36839 |
| 30C | -0.72319 | 0.45473 | 0.97428 |
| 30Ha | -1.10211 | 1.05736 | 1.42741 |
| 30Hb | -1.04123 | -0.18465 | 1.57288 |
| 30Hc | 0.01649 | 0.57635 | 0.91091 |
| 31O | -2.23866 | 0.52138 | -0.85773 |
| 32O | 0.77273 | -0.30633 | -3.53393 |
| 33C | 2.05288 | 0.08000 | -3.30407 |
| 34C | 2.32585 | 1.40712 | -3.91445 |
| 34Ha | 1.81462 | 1.52020 | -4.76910 |
| 34Hb | 3.25586 | 1.62262 | -4.18105 |
| 34Hc | 1.90479 | 2.05754 | -3.25740 |
| 35O | 2.82583 | -0.57067 | -2.67585 |

2. (R)-(+)-3-(1-methyl-2-pyrrolidinyl)-2-pyridyl β-D-glucopyranoside (1b)

Using 4.8g of (R)-(+)-3-(1-methyl-2-pyrrolidinyl)-2-pyridyl 2,3,4,6-tetra-O-acetyl-β-D-glucopyranoside (5b), (R)-(+)-3-(1-methyl-2-pyrrolidinyl)-2-pyridyl β-D-glucopyranoside (1b) (3.0g) was obtained in white solid form in the same manner as in 2 of Example 1.

Physical properties

$[\alpha]_D$ +39.1° (c 0.82, methanol)

UV: $\lambda_{max}$ 223 nm ($\epsilon$ = 2,600), 268 nm ($\epsilon$ = 2,800)

IR: $\nu$ ($cm^{-1}$); 3326, 2926, 1591, 1446, 1251, 1075, 754

$^1H$ - NMR: $\delta$ ($CDCl_3$; ppm from TMS)
1.75 (1H, m),
1.95 (2H, m),
2.36 (3H, s),
2.40 (1H, m),
2.57 (1H, q, J = 9.1 Hz),
3.41 (1H, m),
3.55 (1H, m),
3.4 - 3.6 (4H, m),
3.86 (1H, dd, J = 4.3, 12.0 Hz),
3.99 (1H, m),
5.93 (1H, d, J = 7.4 Hz),
7.12 (1H, dd, J = 5.0, 7.5 Hz),
7.90 (1H, dd, J = 1.7, 7.5 Hz),
8.11 (1H, dd, J = 1.7, 5.0 Hz)

$^{13}C$-NMR: $\delta$ ($CDC\ell_3$; ppm from TMS);
23.2 ($CH_2$), 33.1 ($CH_2$), 40.8 ($CH_3$), 57.7 ($CH_2$), 62.3 ($CH_2$), 65.1 (CH), 71.0 (CH), 74.5 (CH), 78.1 (CH), 78.2 (CH), 97.5 (CH), 119.9 (CH), 125.4 (C), 138.2 (CH), 146.4 (CH), 161.4 (C)

MS (%): 341 ($M^{+1}$) (1.5), 340 (1.4), 235 (5), 179 (90), 178 (16), 177 (43), 163 (81), 149 (19), 148 (18), 84 (100)

HRMS (high resolution mass spectrum) :
Obs. ; 341.1720
cald. for ; $C_{12}H_{25}N_2O_6$ ($M^{+1}$) 341.1713

In the above physical properties, since disappearance of carbonyl absorption (in the vicinity of 1647 $cm^{-1}$) of the compound (4b) in IR, and a characteristic signal of β-O-glycoside ($\delta$ 5.93 (1H, d, J = 7.4 Hz)) in $^1H$ - NMR are observed, the structure of the compound (1b) can be confirmed.

Example 3

Synthesis of (S)-(-)-3-(1-methyl-2-pyrrolidinyl)-2-pyridyl β-D-galactopyranoside (1c)

1. (S)-(-)-3-(1-methyl-2-pyrrolidinyl)-2-pyridyl 2,3,4,6-tetra-O-acetyl-β-D-galactopyranoside (5c)

Using 2g of (S)-(-)-3-(1-methyl-2-pyrrolidinyl)-2-pyridone (4a) and 5.6g of 2,3,4,6-tetra-O-acetylgalactopyranosyl bromide (3b) as starting material, (S)-(-)-3-(1-methyl-2-pyrrolidinyl)-2-pyridyl 2,3,4,6-tetra-O-acetyl-β-D-galactopyranoside (5c)(5.0g) was obtained in white solid form in the same manner as in 1 of Example 1. This was recrystallized from ethyl acetate - hexan to give compound (5c) in high purity (4.9g; 88 %).

Physical properties

$[\alpha]_D$ -40.9° (c 0.57, methanol)

IR: $\nu$ ($cm^{-1}$); 2924, 1750, 1589, 1437, 1371, 1234, 1064

$^1H$ - NMR: $\delta$ ($CDC\ell_3$; ppm from TMS)
1.55 (1H, m),
1.85 (2H, m),

1.97 (3H, s),
2.02 (3H, s),
2.03 (3H, s),
2.15 (3H, s),
2.20 (3H, s),
2.34 (1H, q, J = 9.3 Hz),
2.35 (1H, m),
3.22 (1H, ddd, J = 2.3, 7.1, 9.3 Hz),
3.40 (1H, t, J = 8.3 Hz),
4.17 (3H, m),
5.17 (1H, dd, J = 3.5, 10.5 Hz),
5.48 (1H, d, J = 3.5 Hz),
5.52 (1H, dd, J = 8.3, 10.5 Hz),
6.11 (1H, d, J = 8.3 Hz),
7.02 (1H, dd, J= 4.9, 7.4 Hz),
7.85 (1H, dd, J= 1.9, 7.4 Hz),
8.03 (1H, dd, J= 1.9, 4.9 Hz)

$^{13}$C-NMR: δ (CDCℓ$_3$; ppm from TMS);
20.7 ($CH_3$), 22.9 ($CH_2$), 33.6 ($CH_2$), 40.6 ($CH_3$), 57.0 ($CH_2$), 61.1 ($CH_2$), 63.0 (CH), 67.1 (CH), 68.5 (CH), 71.2 (CH), 94.3 (CH), 119.4 (CH), 126.8 (C), 136.9 (CH), 144.6 (CH), 159.4 (C), 169.5 (C), 170.5 (C), 170.9 (C)

MS (%): 331 (3), 178 (26), 177 (32), 163 (22), 149 (14), 84 (34), 43 (100)

In the above physical properties, since disappearance of carbonyl absorption (in the vicinity of 1647 cm$^{-1}$) of the compound (4a) in IR, a characteristic signal of β-O-glycoside (δ 6.11 (1H, d, J = 8.3 Hz)) in $^1$H - NMR, and signals (1.97 (3H, s), 2.02 (3H, s), 2.03 (3H, s), 2.15 (3H, S) and 2.20 (3H, s)) of acetyl groups and an N-methyl group of (S)-(-)-3-(1-methyl-2-pyrrolidinyl)-2-pyridyl are observed, the structure of the compound (5c) can be confirmed.

2. (S)-(-)-3-(1-methyl-2-pyrrolidinyl)-2-pyridyl β-D-galactopyranoside (1c)

Using 4.9g of (S)-(-)-3-(1-methyl-2-pyrrolidinyl)-2-pyridyl 2,3,4,6-tetra-O-acetyl-β-D-galactopyranoside (5c) as starting material,
(S)-(-)-3-(1-methyl-2-pyrrolidinyl)-2-pyridyl β-D-galactopyranoside (1c)(3.1g) was obtained in white solid form in the same manner as in 2 of Example 1.

Physical properties

$[\alpha]_D$ -48.5° (c 4.9, methanol)

IR: ν (cm$^{-1}$); 3364, 2926, 1593, 1437, 1363, 1243, 1069

$^1$H - NMR: δ (CDCℓ$_3$; ppm from TMS)
1.8 - 2.05 (3H, m),
2.40 (3H, s),
2.40 (1H, m),
2.66 (1H, q, J = 9.8 Hz),
3.38 (1H, m),
3.63 (1H, dd, J = 3.3, 9.7 Hz),
3.74 (3H, m),
3.87 (1H, dd, J = 8.0, 9.7 Hz),
3.95 (2H, m),
5.88 (1H, d, J = 8.0 Hz),
7.09 (1H, dd, J = 5.0, 7.4 Hz),
7.86 (1H, dd, J = 1.8, 7.4 Hz),
8.10 (1H, dd, J = 1.8, 5.0 Hz)

$^{13}$C-NMR: δ (CDCℓ$_3$; ppm from TMS);
22.1 ($CH_2$), 32.7 ($CH_2$) 40.6 ($CH_3$), 57.7 ($CH_2$), 62.1 ($CH_2$), 65.6 (CH), 70.0 (CH), 71.8 (CH), 75.1 (CH), 76.9 (CH), 98.1 (CH), 119.9 (CH), 124.3 (C), 138.6 (CH), 146.8 (CH), 161.4 (C)

MS (%): 340 ($M^+$) (2), 325 (6), 179 (83), 178 (72), 177 (100), 164 (30), 163 (100), 149 (72), 148 (27),

135 (22), 122 (45), 121 (22), 120 (36), 108 (43), 84 (100)

In the above physical properties, since disappearance of carbonyl absorption (in the vicinity of 1647 $cm^{-1}$) of the compound (4a) in IR, and a characteristic signal of β-O-glycoside (δ 5.88 (1H, d, J = 8.0 Hz)) in $^1H$ - NMR are observed, the structure of the compound (1c) can be confirmed.

Example 4

Synthesis of (R)-(+)-3-(1-methyl-2-pyrrolidinyl)-2-pyridyl β-D-galactopyranoside (1d)

1. (R)-(+)-3-(1-methyl-2-pyrrolidinyl)-2-pyridyl 2,3,4,6-tetra-O-acetyl-β-D-galactopyranoside (5d)

Using 2.0g of (R)-(+)-3-(1-methyl-2-pyrrolidinyl)-2-pyridone (4b) and 5.6g of 2,3,4,6-tetra-O-acetylgalactopyranosyl bromide (3b) as starting material,
(R)-(+)-3-(1-methyl-2-pyrrolidinyl)-2-pyridyl 2,3,4,6-tetra-O-acetyl-β-D-galactopyranoside (5d)(4.8g) was obtained in the same manner as in 1 of Example 1.

Physical properties

$[\alpha]_D$ +60.1° (c 0.81, methanol)

IR: ν ($cm^{-1}$); 1748, 1591, 1441, 1373, 1241, 1071

$^1H$ - NMR: δ ($CDCl_3$; ppm from TMS)
1.35 - 1.45 (1H, m),
1.75 - 1.85 (2H, m),
1.97 (3H, s),
2.02 (3H, s),
2.03 (3H, s),
2.15 (3H, s),
2.24 (3H, s),
2.30 (1H, m),
2.37 (1H, q, J = 8.8 Hz),
3.20 (1H, m),
3.51 (1H, t, J = 8.2 Hz),
4.18 (3H, m),
5.17 (1H, dd, J = 3.5, 10.3 Hz),
5.49 (1H, d, J = 3.5 Hz),
5.55 (1H, dd, J = 8.3, 10.3 Hz),
6.19 (1H, d, J = 8.3 Hz),
7.01 (1H, dd, J = 4.9, 7.4 Hz),
7.85 (1H, dd, J = 1.9, 7.4 Hz),
8.01 (1H, dd, J = 1.9, 4.9 Hz)

$^{13}C$-NMR: δ ($CDCl_3$; ppm from TMS);
20.9 ($CH_3$), 23.0 ($CH_2$) 33.3 ($CH_2$), 40.9 ($CH_3$), 57.1 ($CH_2$), 61.2 ($CH_2$), 63.0 (CH), 67.2 (CH), 68.4 (CH), 71.3 (CH), 71.4 (CH), 93.7 (CH), 119.3 (CH), 127.3 (CH), 136.3 (CH), 144.3 (CH), 159.2 (C) 169.6 (C), 170.3 (C), 170.5 (C)

MS (%): 178 (14), 177 (36), 163 (46), 149 (19), 84 (49), 43 (100),

In the above physical properties, since disappearance of carbonyl absorption (in the vicinity of 1647 $cm^{-1}$) of the compound (4b) in IR, a characteristic signal of β-O-glycoside (δ 6.19 (1H, d, J = 8.3 Hz)) in $^1H$ - NMR and signals (1.97 (3H, s), 2.02 (3H, s), 2.03 (3H, s), 2.15 (3H, S) and 2.24 (3H, s)) of acetyl groups and an N-methyl group of (S)-(-)-3-(1-methyl-2-pyrrolidinyl)-2-pyridyl are observed, the structure of the compound (5d) can be confirmed.

2. (R)-(+)-3-(1-methyl-2-pyrrolidinyl)-2-pyridyl β-D-galactopyranoside (1d)

Using 4.8g of (R)-(+)-3-(1-methyl-2-pyrrolidinyl)-2-pyridyl 2,3,4,6-tetra-O-acetyl-β-D-galactopyranoside (5d) as starting material,
(R)-(+)-3-(1-methyl-2-pyrrolidinyl)-2-pyridyl β-D-galactopyranoside (1d)(3.0g) was obtained in white solid form in the same manner as in 2 of Example 1.

Physical properties

$[\alpha]_D$ +31.0° (c 0.98, methanol)
IR: $\nu$ ($cm^{-1}$); 3404, 1598, 1444, 1400, 1071
$^1H$ - NMR: $\delta$ ($CDC\ell_3$; ppm from TMS)
2.00 (1H, m),
2.10 (2H, m),
2.34 (3H, s),
2.35 (1H, m),
2.63 (1H, q, J = 9.5 Hz),
3.40 (1H, m),
3.62 (1H, dd, J = 3.4, 9.8 Hz),
3.75 (4H, m),
3.88 (1H, dd, J = 8.0, 9.8 Hz),
3.93 (1H, d, J = 3.4 Hz),
5.64 (1H, d, J = 8.0 Hz),
7.11 (1H, dd, J = 5.0, 7.4 Hz),
7.79 (1H, dd, J = 1.9, 7.4 Hz),
8.15 (1H, dd, J= 1.9, 5.0 Hz)
$^{13}C$-NMR: $\delta$ ($CDC\ell_3$; ppm from TMS);
23.2 ($CH_2$), 33.1 ($CH_2$) 40.8 ($CH_3$), 57.7 ($CH_2$), 62.3 ($CH_2$), 65.1 (CH), 71.0 (CH), 74.5 (CH), 78.1 (CH), 78.2 (CH), 97.5 (CH), 119.9 (CH), 125.4 (C), 138.2 (CH), 146.4 (CH), 161.4 (C)
MS (%): 340 ($M^+$)(2), 325 (5), 179 (100), 178 (46), 177 (100), 163 (100), 149 (49), 148 (34), 122 (38), 108 (29), 84 (100)

In the above physical properties, since disappearance of carbonyl absorption (in the vicinity of 1647 $cm^{-1}$) of the compound (4b) in IR, and a characteristic signal of $\beta$-O-glycoside ($\delta$ 5.64 (1H, d, J = 8.0 Hz)) in $^1H$ - NMR are observed, the structure of the compound (1d) can be confirmed.

Example 5

Synthesis of (S)-(-)-5-(1-methyl-2-pyrrolidinyl)-2-pyridyl $\beta$-D-glucopyranoside (1e)

1. (S)-(-)-5-(1-methyl-2-pyrrolidinyl)-2-pyridyl 2,3,4,6-tetra-O-acetyl-$\beta$-D-glucopyranoside (5e)

Using 2.0g of (S)-(-)-5-(1-methyl-2-pyrrolidinyl)-2-pyridone (4c) and 5.6g of 2,3,4,6-tetra-O-acetyl-$\beta$-D-glucopyranosyl bromide (3a) as starting material, (S)-(-)-5-(1-methyl-2-pyrrolidinyl)-2-pyridyl 2,3,4,6-tetra-O-acetyl-$\beta$-D-glucopyranoside (5e)(5.1g) was obtained in white solid form in the same manner as in 1 of Example 1. This was recrystallized from ethyl acetate - hexan to give compound (5e) in high purity (5.0g; 88%).

Physical properties

Melting point: 131.0 - 133.0°C
$[\alpha]_D$ -52.5° (c 0.63, methanol)
UV: $\lambda_{max}$ 212 nm ($\epsilon$ = 10,200), 271 nm, ($\epsilon$ = 2,700)
IR: $\nu$ ($cm^{-1}$); 2948, 2782, 1756, 1605, 1485, 1371, 1238, 1040, 909, 733
$^1H$ - NMR: $\delta$ ($CDC\ell_3$; ppm from TMS)
1.72 - 2.2 (4H, m),
1.99 (3H, s),
2.04 (3H, s),
2.05 (6H, s),
2.14 (3H, s),
2.28 (1H, q, J = 7.6 Hz),
3.03 (1H, t, J = 8.2 Hz),
3.22 (1H, t, J = 7.6 Hz),
3.95 (1H, ddd, J = 2.1, 4.2, 9.3 Hz),
4.13 (1H, dd, J = 2.1, 12.4 Hz),

4.32 (1H, dd, J = 4.2, 12.4 Hz),
5.21 (1H, t, J = 9.3 Hz),
5.34 (2H, m),
6.17 (1H, d, J = 7.3 Hz),
6.79 (1H, d, J = 8.5 Hz),
7.66 (1H, dd, J = 2.3, 8.5 Hz),
8.04 (1H, d, J = 2.3 Hz)

$^{13}$C-NMR: δ ($CDC\ell_3$; ppm from TMS);
20.8 ($CH_3$), 22.6 ($CH_2$), 35.2 ($CH_2$), 40.4 ($CH_3$), 57.1 ($CH_2$), 61.8 ($CH_2$), 68.3 (CH), 71.0 (CH), 72.3 (CH), 73.3 (CH), 93.7 (CH), 111.9 (CH), 133.9 (C), 138.7 (CH), 146.1 (CH), 160.9 (C), 169.6 (C), 170.4 (C), 170.8 (C)

MS (%): 509 ($M^{+1}$) (36), 508 (53), 507 (27), 331 (35), 179 (35), 178 (46), 177 (85), 169 (100), 150 (26), 149 (47), 127 (33), 109 (94)

X-ray diffraction data of compound (5e)

bond distance

| | | | | | |
|---|---|---|---|---|---|
| 1N - 2C | 1.455 | 6C - 6Hb | 1.091 | 13C - 13H | 1.017 |
| 1N - 5C | 1.488 | 6C - 6Hc | 0.949 | 14C - 15C | 1.523 |
| 1N - 6C | 1.482 | 7N - 8C | 1.360 | 14C - 24O | 1.435 |
| 2C - 3C | 1.521 | 7N - 12C | 1.338 | 14C - 14H | 0.999 |
| 2C - 9C | 1.500 | 8C - 9C | 1.355 | 15C - 16C | 1.511 |
| 2C - 2H | 1.023 | 8C - 8H | 0.960 | 15C - 28O | 1.440 |
| 3C - 4C | 1.518 | 9C - 10C | 1.387 | 15C - 15H | 0.874 |
| 3C - 3Ha | 0.967 | 10C - 11C | 1.368 | 16C - 17C | 1.515 |
| 3C - 3Hb | 0.908 | 10C - 10H | 0.935 | 16C - 32O | 1.444 |
| 4C - 5C | 1.524 | 11C - 12C | 1.337 | 16C - 16H | 0.987 |
| 4C - 4Ha | 1.097 | 11C - 11H | 0.924 | 17C - 18O | 1.434 |
| 4C - 4Hb | 0.947 | 12C - 36O | 1.378 | 17C - 19C | 1.510 |
| 5C - 5Ha | 0.888 | 13C - 36O | 1.407 | 17C - 17H | 1.013 |
| 5C - 5Hb | 0.844 | 13C - 18O | 1.420 | 19C - 20O | 1.451 |
| 6C - 6Ha | 1.060 | 13C - 14C | 1.518 | 19C - 19Ha | 1.006 |
| 19C - 19Hb | 0.986 | 25C - 27O | 1.195 | 30C - 30Hc | 1.044 |

| | | | | | |
|---|---|---|---|---|---|
| $^{20}O$ - $^{21}C$ | 1.329 | $^{26}C$ - $^{26}Ha$ | 0.949 | $^{32}O$ - $^{33}C$ | 1.353 |
| $^{21}C$ - $^{22}C$ | 1.475 | $^{26}C$ - $^{26}Hb$ | 0.952 | $^{33}C$ - $^{34}C$ | 1.489 |
| $^{21}C$ - $^{23}O$ | 1.179 | $^{26}C$ - $^{26}Hc$ | 0.898 | $^{33}C$ - $^{35}O$ | 1.187 |
| $^{22}C$ - $^{22}Ha$ | 1.060 | $^{28}O$ - $^{29}C$ | 1.355 | $^{34}C$ - $^{34}Ha$ | 1.042 |
| $^{22}C$ - $^{22}Hb$ | 0.925 | $^{29}C$ - $^{30}C$ | 1.479 | $^{34}C$ - $^{34}Hb$ | 0.845 |
| $^{22}C$ - $^{22}Hc$ | 1.015 | $^{29}C$ - $^{31}O$ | 1.191 | $^{34}C$ - $^{34}Hc$ | 0.925 |
| $^{24}O$ - $^{25}C$ | J1.348 | $^{30}C$ - $^{30}Ha$ | 0.927 | | |
| $^{25}C$ - $^{26}C$ | 1.485 | $^{30}C$ - $^{30}Hb$ | 0.904 | | |

bond angle

| | | | | | |
|---|---|---|---|---|---|
| $^{2}C-^{1}N-^{5}C$ | 103.3 | $^{4}C-^{5}C-^{1}N$ | 103.7 | $^{11}C-^{10}C-^{10}H$ | 122.80 |
| $^{2}C-^{1}N-^{6}C$ | 113.2 | $^{4}C-^{5}C-^{5}Ha$ | 112.10 | $^{10}C-^{11}C-^{12}C$ | 118.5 |
| $^{5}C-^{1}N-^{6}C$ | 113.7 | $^{4}C-^{5}C-^{5}Hb$ | 119.61 | $^{10}C-^{11}C-^{11}H$ | 115.00 |
| $^{1}N-^{2}C-^{3}C$ | 103.4 | $^{1}N-^{5}C-^{5}Ha$ | 86.56 | $^{12}C-^{11}C-^{11}H$ | 126.46 |
| $^{1}N-^{2}C-^{9}C$ | 111.8 | $^{1}N-^{5}C-^{5}Hb$ | 107.23 | $^{11}C-^{12}C-^{7}N$ | 124.0 |
| $^{1}N-^{2}C-^{2}H$ | 109.22 | $^{5}Ha-^{5}C-^{5}Hb$ | 120.24 | $^{11}C-^{12}C-^{36}O$ | 124.2 |
| $^{3}C-^{2}C-^{9}C$ | 115.5 | $^{1}N-^{6}C-^{6}Ha$ | 108.22 | $^{7}N-^{12}C-^{36}O$ | 111.8 |
| $^{3}C-^{2}C-^{2}H$ | 108.03 | $^{1}N-^{6}C-^{6}Hb$ | 86.35 | $^{12}C-^{36}O-^{13}C$ | 116.7 |
| $^{9}C-^{2}C-^{2}H$ | 108.68 | $^{1}N-^{6}C-^{6}Hc$ | 110.65 | $^{14}C-^{13}C-^{18}O$ | 107.7 |
| $^{2}C-^{3}C-^{4}C$ | 105.7 | $^{6}Ha-^{6}C-^{6}Hb$ | 114.37 | $^{14}C-^{13}C-^{36}O$ | 107.2 |
| $^{2}C-^{3}C-^{3}Ha$ | 112.30 | $^{6}Ha-^{6}C-^{6}Hc$ | 112.49 | $^{14}C-^{13}C-^{13}H$ | 115.96 |
| $^{2}C-^{3}C-^{3}Hb$ | 105.84 | $^{6}Hb-^{6}C-^{6}Hc$ | 121.02 | $^{18}O-^{13}C-^{36}O$ | 107.2 |
| $^{4}C-^{3}C-^{3}Ha$ | 105.67 | $^{8}C-^{7}N-^{12}C$ | 116.4 | $^{18}O-^{13}C-^{13}H$ | 107.54 |
| $^{4}C-^{3}C-^{3}Hb$ | 119.14 | $^{7}N-^{8}C-^{9}C$ | 123.8 | $^{36}O-^{13}C-^{13}H$ | 111.16 |
| $^{3}Ha-^{3}C-^{3}Hb$ | 108.27 | $^{7}N-^{8}C-^{8}H$ | 114.99 | $^{13}C-^{14}C-^{15}C$ | 109.2 |
| $^{3}C-^{4}C-^{5}C$ | 104.3 | $^{9}C-^{8}C-^{8}H$ | 121.11 | $^{13}C-^{14}C-^{24}O$ | 107.5 |
| $^{3}C-^{4}C-^{4}Ha$ | 117.79 | $^{8}C-^{9}C-^{10}C$ | 116.8 | $^{13}C-^{14}C-^{14}H$ | 109.54 |

| | | | | | |
|---|---|---|---|---|---|
| $^{3}C-^{4}C-^{4}Hb$ | 118.36 | $^{8}C-^{9}C-^{2}C$ | 121.8 | $^{15}C-^{14}C-^{24}O$ | 110.1 |
| $^{5}C-^{4}C-^{4}Ha$ | 105.48 | $^{2}C-^{9}C-^{10}C$ | 121.4 | $^{15}C-^{14}C-^{14}H$ | 108.40 |
| $^{5}C-^{4}C-^{4}Hb$ | 99.93 | $^{9}C-^{10}C-^{11}C$ | 120.5 | $^{24}O-^{14}C-^{14}H$ | 112.07 |
| $^{4}Ha-^{4}C-^{4}Hb$ | 108.42 | $^{9}C-^{10}C-^{10}H$ | 116.65 | $^{14}C-^{15}C-^{16}C$ | 110.6 |
| $^{14}C-^{15}C-^{28}O$ | 107.3 | $^{20}O-^{19}C-^{19}Hb$ | 114.80 | $^{15}C-^{28}O-^{29}C$ | 119.22 |
| $^{14}C-^{15}C-^{15}H$ | 106.86 | $^{19}Ha-^{19}C-^{19}Hb$ | 110.26 | $^{28}O-^{29}C-^{30}C$ | 110.56 |
| $^{16}C-^{15}C-^{28}O$ | 107.3 | $^{19}C-^{20}O-^{21}C$ | 117.98 | $^{28}O-^{29}C-^{31}O$ | 123.84 |
| $^{16}C-^{15}C-^{15}H$ | 110.03 | $^{20}O-^{21}C-^{22}C$ | 109.49 | $^{30}C-^{29}C-^{31}O$ | 125.54 |
| $^{28}O-^{15}C-^{15}H$ | 113.18 | $^{20}O-^{21}C-^{23}O$ | 123.65 | $^{29}C-^{30}C-^{30}Ha$ | 120.02 |
| $^{15}C-^{16}C-^{17}C$ | 112.1 | $^{22}C-^{21}C-^{23}O$ | 126.86 | $^{29}C-^{30}C-^{30}Hb$ | 107.27 |
| $^{15}C-^{16}C-^{32}O$ | 108.0 | $^{21}C-^{22}C-^{22}Ha$ | 106.81 | $^{29}C-^{30}C-^{30}Hc$ | 107.90 |
| $^{15}C-^{16}C-^{16}H$ | 108.40 | $^{21}C-^{22}C-^{22}Hb$ | 102.81 | $^{30}Ha-^{30}C-^{30}Hb$ | 89.53 |
| $^{17}C-^{16}C-^{32}O$ | 107.0 | $^{21}C-^{22}C-^{22}Hc$ | 98.33 | $^{30}Ha-^{30}C-^{30}Hc$ | 111.80 |
| $^{17}C-^{16}C-^{16}H$ | 109.36 | $^{22}Ha-^{22}C-^{22}Hb$ | 131.04 | $^{30}Hb-^{30}C-^{30}Hc$ | 120.04 |
| $^{32}O-^{16}C-^{16}H$ | 111.99 | $^{22}Ha-^{22}C-^{22}Hc$ | 98.38 | $^{16}C-^{32}O-^{33}C$ | 117.35 |
| $^{16}C-^{17}C-^{19}C$ | 113.1 | $^{22}Hb-^{22}C-^{22}Hc$ | 114.91 | $^{32}O-^{33}C-^{34}C$ | 110.98 |
| $^{16}C-^{17}C-^{18}O$ | 109.9 | $^{14}C-^{24}O-^{25}C$ | 117.80 | $^{32}O-^{33}C-^{35}O$ | 123.14 |
| $^{16}C-^{17}C-^{17}H$ | 107.93 | $^{24}O-^{25}C-^{26}C$ | 112.79 | $^{34}C-^{33}C-^{35}O$ | 125.87 |

| | | | | | |
|---|---|---|---|---|---|
| $^{19}C-^{17}C-^{18}O$ | 107.0 | $^{24}O-^{25}C-^{27}O$ | 121.96 | $^{33}C-^{34}C-^{34}Ha$ | 110.93 |
| $^{19}C-^{17}C-^{17}H$ | 108.75 | $^{26}C-^{25}C-^{27}O$ | 125.14 | $^{33}C-^{34}C-^{34}Hb$ | 118.49 |
| $^{18}O-^{17}C-^{17}H$ | 110.18 | $^{25}C-^{26}C-^{26}Ha$ | 107.10 | $^{33}C-^{34}C-^{34}Hc$ | 103.30 |
| $^{17}C-^{18}O-^{13}C$ | 111.20 | $^{25}C-^{26}C-^{26}Hb$ | 114.10 | $^{34}Ha-^{34}C-^{34}Hb$ | 102.99 |
| $^{17}C-^{19}C-^{20}O$ | 108.27 | $^{25}C-^{26}C-^{26}Hc$ | 107.77 | $^{34}Ha-^{34}C-^{34}Hc$ | 105.54 |
| $^{17}C-^{19}C-^{19}Ha$ | 103.96 | $^{26}Ha-^{26}C-^{26}Hb$ | 134.88 | $^{34}Hb-^{34}C-^{34}Hc$ | 115.07 |
| $^{17}C-^{19}C-^{19}Hb$ | 106.47 | $^{26}Ha-^{26}C-^{26}Hc$ | 105.44 | | |
| $^{20}O-^{19}C-^{19}Ha$ | 112.30 | $^{26}Hb-^{26}C-^{26}Hc$ | 79.16 | | |

Cartesian co-ordinaates

| | x | y | z |
|---|---|---|---|
| $^{1}$N | -4.22058 | -8.33454 | -1.70886 |
| $^{2}$C | -3.31343 | -7.20563 | -1.96711 |
| $^{2}$H | -3.60017 | -6.47150 | -1.38056 |
| $^{3}$C | -1.95691 | -7.71856 | -1.57675 |
| $^{3}$Ha | -1.13262 | -7.02626 | -1.21478 |
| $^{3}$Hb | -1.48396 | -8.12579 | -2.28619 |
| $^{4}$C | -5.65585 | -7.97544 | -1.69981 |
| $^{4}$Ha | -2.06476 | -8.59573 | 0.35995 |
| $^{4}$Hb | -2.00239 | -9.51595 | -0.66734 |
| $^{5}$C | -3.76654 | -8.92185 | -0.49532 |
| $^{5}$Ha | -4.09759 | -9.97525 | -0.32242 |
| $^{5}$Hb | -3.93822 | -8.31384 | 0.42148 |
| $^{6}$C | -5.65585 | -7.97544 | -1.69981 |
| $^{6}$Ha | -6.40202 | -8.78819 | -1.62272 |
| $^{6}$Hb | -5.29985 | -7.30632 | -0.79356 |
| $^{6}$Hc | -6.17900 | -7.19102 | -2.73536 |
| $^{7}$N | -2.92873 | -5.04631 | -5.02542 |
| $^{8}$C | -2.86954 | -5.53211 | -3.79869 |
| $^{3}$C | -3.41628 | -6.74670 | -3.39268 |
| $^{10}$C | -4.00703 | -7.48709 | -4.38006 |
| $^{10}$H | -4.41207 | -8.15052 | -4.30385 |
| $^{11}$C | -4.06632 | -7.02193 | -5.66225 |
| $^{11}$H | -4.41635 | -7.42324 | -6.51708 |
| $^{12}$C | -3.53161 | -5.80282 | -5.94450 |
| $^{12}$H | -3.95444 | -5.44880 | -6.86947 |
| $^{36}$O | -2.20968 | -4.80445 | -2.83126 |
| $^{13}$C | -1.72340 | -3.53500 | -3.17644 |
| $^{13}$H | -2.31435 | -2.99915 | -3.78234 |
| $^{14}$C | -1.49013 | -2.79547 | -1.89934 |
| $^{14}$H | -0.96157 | -3.32708 | -1.30786 |
| $^{15}$C | -0.86930 | -1.42591 | -2.19653 |
| $^{15}$H | -1.51246 | -0.82048 | -2.64720 |
| $^{16}$C | 0.37647 | -1.60377 | -3.05154 |
| $^{15}$H | 1.07468 | -2.03669 | -2.54453 |

| | | | |
|---|---|---|---|
| 17C | 0.08200 | -2.48640 | -4.27036 |
| 17H | -0.55250 | -1.95846 | -4.81745 |
| 18O | -0.46321 | -3.72926 | -3.80774 |
| 19C | 1.32063 | -2.79661 | -5.05574 |
| 19Ha | 1.00493 | -3.39728 | -5.77028 |
| 19Hb | 1.60758 | -2.00338 | -5.41200 |
| 20O | 2.25890 | -3.43765 | -4.18105 |
| 21C | 3.56827 | -3.36834 | -4.49740 |
| 22C | 4.37048 | -4.08884 | -3.46159 |
| 22Ha | 4.26074 | -4.92279 | -3.60082 |
| 22Hb | 4.19566 | -3.43189 | -2.48942 |
| 22Hc | 4.88364 | -4.03171 | -3.70033 |
| 23O | 3.97633 | -2.81352 | -5.43486 |
| 24O | -2.74804 | -2.57547 | -1.24440 |
| 25C | -2.86067 | -2.95438 | 0.03059 |
| 26C | -4.19138 | -2.64842 | 0.60475 |
| 26Ha | -4.52472 | -1.71580 | 0.15910 |
| 26Hb | -4.34747 | -3.02933 | 1.27086 |
| 26Hc | -4.78189 | -3.28925 | 0.30369 |
| 27O | -1.96606 | -3.41190 | 0.61890 |
| 28O | -0.45284 | -0.82974 | -0.95257 |
| 29C | -1.24331 | 0.09809 | -0.36839 |
| 30C | -0.72319 | 0.45473 | 0.97428 |
| 30Ha | -1.10211 | 1.05736 | 1.42741 |
| 30Hb | -1.04123 | -0.18465 | 1.57288 |
| 30Hc | 0.01649 | 0.57635 | 0.91091 |
| 31O | -2.23866 | 0.52138 | -0.85773 |
| 32O | 0.77273 | -0.30633 | -3.53393 |
| 33C | 2.05288 | 0.08000 | -3.30407 |
| 34C | 2.32585 | 1.40712 | -3.91445 |
| 34Ha | 1.81462 | 1.52020 | -4.76910 |
| 34Hb | 3.25586 | 1.62262 | -4.18105 |
| 34Hc | 1.90479 | 2.05754 | -3.25740 |
| 35O | 2.82583 | -0.57067 | -2.67585 |

In the above physical properties, since disappearance of carbonyl absorption (in the vicinity of 1661 $cm^{-1}$) of the compound (4c) in IR, a characteristic signal of $\beta$-O-glycoside ($\delta$ 6.17 (1H, d, J = 7.3 Hz)) in $^1H$ - NMR, and signals ($\delta$ 1.99 (3H, s), 2.04 (3H, s), 2.05 (6H, s)) of acetyl groups are observed, and from the X-ray crystal structure analysis, the structure of the compound (5e) can be confirmed.

2. (S)-(-)-5-(1-methyl-2-pyrrolidinyl)-2-pyridyl β-D-glucopyranoside (1e)

Using 5.0g of (S)-(-)-5-(1-methyl-2-pyrrolidinyl)-2-pyridyl 2,3,4,6-tetra-O-acetyl-β-D-glucopyranoside (5e), (S)-(-)-5-(1-methyl-2-pyrrolidinyl)-2-pyridyl β-D-glucopyranoside (1e)(3.0g) was obtained in white solid form in the same manner as in 2 of Example 1.

Physical properties

$[\alpha]_D$ -79.7° (c 0.73, methanol)
UV: $\lambda_{max}$ 215 nm (ε = 8,400), 271 nm, (ε = 2,800)
IR: ν ($cm^{-1}$); 3280, 2970, 1607, 1491, 1253, 1077
$^1H$ - NMR: δ ($CDCl_3$; ppm from TMS)
1.75 - 1.95 (3H, m),
2.19 (3H, s),
2.20 (1H, m),
2.39 (1H, q, J = 9.1 Hz),
3.18 (1H, t, J = 7.5 Hz),
3.25 (1H, m),
3.4 - 3.5 (4H, m),
3.69 (1H, dd, J = 4.8, 12.1 Hz),
3.86 (1H, dd, J = 2.0, 12.1 Hz),
5.67 (1H, d, J = 7.7 Hz),
6.94 (1H, d, J = 8.5 Hz),
7.78 (1H, dd, J = 2.4, 8.5 Hz),
8.09 (1H, d, J = 2.4 Hz)
$^{13}C$-NMR: δ ($CDCl_3$; ppm from TMS);
22.6 ($CH_2$), 34.1 ($CH_2$) 40.0 ($CH_3$), 57.3 ($CH_2$), 61.7 ($CH_2$), 69.1 (CH), 70.5 (CH), 73.9 (CH), 76.8 (CH), 77.5 (CH), 98.2 (CH), 112.5 (CH), 132.6 (C), 140.9 (CH), 147.2 (CH), 162.9 (C)
MS (%): 340 ($M^{+1}$) (2), 207 (3), 179 (20), 178 (66), 177 (75), 150 (25), 149 (79), 136 (13), 135 (25), 122 (14), 121 (16), 108 (11), 85 (11), 84 (100), 60 (31), 57 (26)
HRMS (high resolution mass spectrum):
Obs. ; 341.1720
cald. for ; $C_{12}H_{25}N_2O_6$ ($M^{+1}$) 341.1713.

In the above physical properties, since disappearance of carbonyl absorption (in the vicinity of 1661 $cm^{-1}$) of the compound (4c) in IR, a characteristic signal of β-O-glycoside (δ 5.67 (1H, d, J = 7.7 Hz)) in $^1H$ - NMR are observed, the structure of the compound (1e) can be confirmed.

Example 6

Synthesis of (R)-(+)-5-(1-methyl-2-pyrrolidinyl)-2-pyridyl β-D-glucopyranoside (1f)

1. (R)-(+)-5-(1-methyl-2-pyrrolidinyl)-2-pyridyl 2,3,4,6-tetra-O-acetyl-β-D-glucopyranoside (5f)

Using 2.0g of (R)-(+)-5-(1-methyl-2-pyrrolidinyl)-2-pyridone (4d) and 5.6g of 2,3,4,6-tetra-O-acetylglucopyranosyl bromide (3a) as starting material, (R)-(-)-5-(1-methyl-2-pyrrolidinyl)-2-pyridyl 2,3,4,6-tetra-O-acetyl-β-D-glucopyranoside (5f)(4.8g) was obtained in the same manner as in 1 of Example 1.

Physical properties

Melting point: 152.0 - 153.0°C
$[\alpha]_D$ +32.4° (c 0.74, methanol)
UV: $\lambda_{max}$ 213 nm (ε = 8,200), 271 nm, (ε = 2,600)
IR: ν ($cm^{-1}$); 2948, 2782, 1756, 1605, 1485, 1369 1236, 1040, 909
$^1H$ - NMR: δ ($CDCl_3$; ppm from TMS)
1.7 - 2.2 (4H, m),

1.99 (3H, s),
2.04 (3H, s),
2.05 (6H, s),
2.15 (3H, s),
2.29 (1H, q, J = 7.6 Hz),
3.03 (1H, t, J = 8.3 Hz),
3.23 (1H, t, J = 7.6 Hz),
3.94 (1H, ddd, J = 2.3, 4.2, 9.8 Hz),
4.12 (1H, dd, J = 2.3, 12.4 Hz),
4.31 (1H, dd, J = 4.3, 12.4 Hz),
5.21 (1H, t, J = 9.8 Hz),
5.33 (2H, m),
6.18 (1H, d, J = 7.7 Hz)
6.79 (1H, d, J = 8.5 Hz),
7.66 (1H, dd, J = 2.3, 8.5 Hz),
8.04 (1H, d, J = 2.3 Hz)

$^{13}$C-NMR: δ (CDCℓ$_3$; ppm from TMS);
20.8 ($CH_3$), 22.7 ($CH_2$), 35.2 ($CH_2$), 40.4 ($CH_3$), 57.1 ($CH_2$), 61.8 ($CH_2$), 68.4 (CH), 71.0 (CH), 72.3 (CH), 73.3 (CH), 93.8 (CH), 111.9 (CH), 134.0 (C), 138.7 (CH), 146.0 (CH), 160.9 (C), 169.6 (C), 170.4 (C), 170.8 (C)

MS (%): 509 ($M^{+1}$) (12), 508 (13), 507 (7), 331 (3), 178 (17), 177 (25), 169 (44), 149 (26), 109 (37), 84 (41), 43 (100)

In the above physical properties, since disappearance of carbonyl absorption (in the vicinity of 1661 $cm^{-1}$) of the compound (4d) in IR, and a characteristic signal of β-O-glycoside (δ 6.18 (1H, d, J = 7.7 Hz)) in $^1$H - NMR, and signals (δ 1.99 (3H, s), 2.04 (3H, s), 2.05 (6H, s)) of acetyl groups are observed, the structure of the compound (5f) can be confirmed.

2. (R)-(+)-5-(1-methyl-2-pyrrolidinyl)-2-pyridyl β-D-glucopyranoside (1f)

Using 4.8g of (R)-(+)-5-(1-methyl-2-pyrrolidinyl)-2-pyridyl 2,3,4,6-tetra-O-acetyl-β-D-glucopyranoside (5f), (R)-(+)-5-(1-methyl-2-pyrrolidinyl)-2-pyridyl β-D-glucopyranoside (1f)(3.0g) was obtained in white solid form in the same manner as in 2 of Example 1.

Physical properties

$[\alpha]_D$ +32.4° (c 0.76, methanol)
UV: $\lambda_{max}$ 215 nm (ε = 9,700), 272 nm, (ε = 3,600)
IR: ν ($cm^{-1}$); 3366, 2922, 1607, 1491, 1338, 1280, 1077
$^1$H - NMR: δ (CDCℓ$_3$; ppm from TMS)
1.70 - 2.00 (3H, m),
2.16 (3H, s),
2.20 (1H, m),
2.36 (1H, q, J = 9.1 Hz),
3.14 (1H, t, J = 7.4 Hz),
3.23 (1H, m),
3.4 - 3.5 (4H, m),
3.69 (1H, dd, J = 4.9, 12.0 Hz),
3.85 (1H, dd, J = 2.1, 12.0 Hz),
5.66 (1H, d, J = 7.7 Hz),
6.94 (1H, d, J = 8.6 Hz),
7.77 (1H, dd, J = 2.4, 8.6 Hz),
8.07 (1H, d, J = 2.4 Hz)

$^{13}$C-NMR: δ (CDCℓ$_3$; ppm from TMS);
22.9 ($CH_2$), 34.1 ($CH_2$), 40.4 ($CH_3$), 57.7 ($CH_2$), 62.4 ($CH_2$), 69.5 (CH), 71.5 (CH), 74.4 (CH), 77.9 (CH), 78.2 (CH), 98.3 (CH), 112.5 (CH), 133.3 (C), 140.1 (CH), 147.3 (CH), 163.5 (C)

MS (%): 340 ($M^{+1}$) (2), 252 (2), 179 (16), 178 (68), 177 (71), 150 (25), 149 (86), 135 (24), 84 (100), 73 (23), 60 (36), 57 (25), 55 (24)

In the above physical properties, since disappearance of carbonyl absorption (in the vicinity of 1661 $cm^{-1}$) of the compound (4d) in IR, and a characteristic signal of β-O-glycoside (δ 5.66 (1H, d, J = 7.7 Hz)) in $^1H$ - NMR are observed, the structure of the compound (1f) can be confirmed.

Example 7

Synthesis of (S)-(-)-5-(1-methyl-2-pyrrolidinyl)-2-pyridyl β-D-galactopyranoside (1g)

1. (S)-(-)-5-(1-methyl-2-pyrrolidinyl)-2-pyridyl 2,3,4,6-tetra-O-acetyl-β-D-galactopyranoside (5g)

Using 2.0g of (S)-(-)-5-(1-methyl-2-pyrrolidinyl)-2-pyridone (4c) and 5.6g of 2,3,4,6-tetra-O-acetyl-D-galactopyranosyl bromide (3b) as starting material, (S)-(-)-5-(1-methyl-2-pyrrolidinyl)-2-pyridyl 2,3,4,6-tetra-O-acetyl-β-D-galactopyranoside (5g)(5.1g) was obtained in white solid form in the same manner as in 1 of Example 1. This was recrystallized from ethyl acetate - hexan to give compound (5g) in high purity (5.0g; 88 %).

Physical properties

$[\alpha]_D$ -31.5° (c 0.67, methanol)

IR: ν ($cm^{-1}$); 2946, 1750, 1605, 1487, 1373, 1236, 1077, 907, 735

$^1H$ - NMR: δ ($CDC\ell_3$; ppm from TMS)
1.7 - 2.2 (4H, m),
1.99 (3H, s),
2.01 (3H, s),
2.02 (3H, s),
2.15 (3H, s),
2.18 (3H, s)
2.28 (1H, q, J = 9.0 Hz)
3.03 (1H, t, J = 8.3 Hz),
3.22 (1H, m),
4.16 (3H, m),
5.17 (1H, dd, J = 3.4, 10.2 Hz),
5.47 (1H, dd, J = 3.2 Hz),
5.51 (1H, dd, J = 8.8, 10.2 Hz),
6.15 (1H, d, J = 8.8 Hz),
6.81 (1H, d, J = 8.5 Hz),
7.66 (1H, dd, J = 2.3, 8.5 Hz),
8.04 (1H, d, J = 2.3 Hz)

$^{13}C$-NMR: δ ($CDC\ell_3$; ppm from TMS);
20.8 ($CH_3$), 22.7 ($CH_2$), 35.2 ($CH_2$), 40.5 ($CH_3$), 57.1 ($CH_2$), 61.2 ($CH_2$), 67.2 (CH), 68.4 (CH), 68.6 (CH), 71.3 (CH), 94.3 (CH), 111.9 (CH), 134.0 (C), 138.7 (CH), 146.1 (CH), 161.0 (C), 169.7 (C), 170.3 (C), 170.5 (C), 170. 9 (C)

MS (%): 508 ($M^{+1}$) (17), 331 (12), 179 (11), 178 (25), 177 (37), 169 (49), 149 (24), 109 (36), 84 (48), 43 (100)

In the above physical properties, since disappearance of carbonyl absorption (in the vicinity of 1661 $cm^{-1}$) of the compound (4c) in IR, a characteristic signal of β-O-glycoside (δ 6.15 (1H, d, J = 8.8 Hz)) in $^1H$ - NMR, and signals (1.99 (3H, s), 2.01 (3H, s), 2.02 (3H, s), 2.15 (3H, S) and 2.18 (3H, s)) of acetyl groups and an N-methyl group of (S)-(-)-5-(1-methyl-2-pyrrolidinyl)-2-pyridyl are observed, the structure of the compound (5g) can be confirmed.

2. (S)-(-)-5-(1-methyl-2-pyrrolidinyl)-2-pyridyl β-D-galactopyranoside (1g)

Using 5.0g of (S)-(-)-5-(1-methyl-2-pyrrolidinyl)-2-pyridyl 2,3,4,6-tetra-O-acetyl-β-D-galactopyranoside (5g), (S)-(-)-3-(1-methyl-2-pyrrolidinyl)-2-pyridyl β-D-galactopyranoside (1g)(3.0g) was obtained in white solid form in the same manner as in 2 of Example 1.

Physical properties

$[\alpha]_D$ -55.2° (c 0.84, methanol)
IR: $\nu$ ($cm^{-1}$); 3370, 1606, 1491, 1075
$^1H$ - NMR: $\delta$ ($CDC\ell_3$; ppm from TMS)
1.85 - 2.10 (3H, m),
2.30 (3H, s),
2.30 (1H, m),
2.58 (1H, q, J = 9.8 Hz)
3.36 (1H, m),
3.44 (1H, m),
3.62 (1H, dd, J = 3.4, 9.7 Hz),
3.74 (3H, m),
3.84 (1H, dd, J = 8.0, 9.7 Hz),
3.94 (1H, d, J = 3.4 Hz),
5.65 (1H, d, J = 8.0 Hz),
6.98 (1H, d, J = 8.6 Hz),
7.83 (1H, dd, J = 2.4, 8.6 Hz),
8.14 (1H, d, J = 2.4 Hz)
$^{13}C$-NMR: $\delta$ ($CDC\ell_3$; ppm from TMS);
22.7 ($CH_2$), 34.0 ($CH_2$) 39.9 ($CH_3$), 57.3 ($CH_2$), 62.2 ($CH_2$), 69.8 (CH), 70.0 (CH), 71.8 (CH), 74.8 (CH), 76.9 (CH), 98.8 (CH), 112.6 (CH), 130.6 (C), 140.4 (CH), 147.8 (CH), 163.9 (C)
MS (%): 340 ($M^{+1}$) (1), 207 (2), 179 (10), 178 (62), 177 (66), 150 (23), 149 (87), 135 (26), 84 (100), 55 (26)

In the above physical properties, since disappearance of carbonyl absorption (in the vicinity of 1661 $cm^{-1}$) of the compound (4c) in IR, a characteristic signal of $\beta$-O-glycoside ($\delta$ 5.65 (1H, d, J = 8.0 Hz) in $^1H$ - NMR are observed, the structure of the compound (1g) can be confirmed.

Example 8

Synthesis of (R)-(+)-5-(1-methyl-2-pyrrolidinyl)-2-pyridyl $\beta$-D-galactopyranoside (1h)

1. (R)-(+)-5-(1-methyl-2-pyrrolidinyl)-2-pyridyl 2,3,4,6-tetra-O-acetyl-$\beta$-D-galactopyranoside (5h)

Using 2.0g of (R)-(+)-5-(1-methyl-2-pyrrolidinyl)-2-pyridone (4d) and 5.6g of 2,3,4,6-tetra-O-acetyl-D-galactopyranosyl bromide (3b) as starting material,
(R)-(+)-5-(1-methyl-2-pyrrolidinyl)-2-pyridyl 2,3,4,6-tetra-O-acetyl-$\beta$-D-galactopyranoside (5h)(4.8g) was obtained in the same manner as in 1 of Example 1.

Physical properties

$[\alpha]_D$ +50.4° (c 1.8, methanol)
IR: $\nu$ ($cm^{-1}$); 2784, 1752, 1605, 1485, 1373, 1236, 1075
$^1H$ - NMR: $\delta$ ($CDC\ell_3$; ppm from TMS)
1.65 - 2.20 (4H, m),
1.99 (3H, s),
2.01 (3H, s),
2.02 (3H, s)
2.15 (3H, s),
2.18 (3H, s),
2.29 (1H, q, J = 9.3 Hz),
3.03 (1H, t, J = 8.3 Hz),
3.23 (1H, m)
4.16 (3H, m),
5.17 (1H, dd, J = 3.4, 10.4 Hz),
5.47 (1H, d, J = 3.4 Hz),
5.51 (1H, dd, J = 8.3, 10.4 Hz),

6.16 (1H, d, J = 8.3 Hz),
6.80 (1H, d, J = 8.4 Hz),
7.67 (1H, dd, J 2.3, 8.4 Hz),
8.04 (1H, d, J = 2.3 Hz)

$^{13}$C-NMR: δ (CDCℓ$_3$; ppm from TMS);
20.8 ($CH_3$), 22.7 ($CH_2$), 35.1 ($CH_2$), 40.5 ($CH_3$), 57.1 ($CH_2$), 61.2 ($CH_2$), 67.2 (CH), 68.4 (CH), 68.6 (CH), 71.2 (CH), 94.2 (CH), 111.9 (CH), 133.9 (C), 138.7 (CH), 146.0 (CH), 161.0 (C), 169.7 (C), 170.5 (C), 170.8 (C)

MS (%): 508 ($M^{+1}$) (1), 331 (8), 178 (12), 177 (16), 169 (24), 127 (12), 109 (18), 84 (13), 43 (100)

In the above physical properties, since disappearance of carbonyl absorption (in the vicinity of 1661 $cm^{-1}$) of the compound (4d) in IR, and a characteristic signal of β-O-glycoside (δ 6.16 (1H, d, J = 8.3 Hz)) in $^1$H - NMR, and signals (1.99 (3H, s), 2.01 (3H, s), 2.02 (3H, s), 2.15 (3H, S) and 2.18 (3H, s)) of acetyl groups and an N-methyl group of (S)-(-)-5-(1-methyl-2-pyrrolidinyl)-2-pyridyl are observed, the structure of the compound (5h) can be confirmed.

2. (R)-(+)-5-(1-methyl-2-pyrrolidinyl)-2-pyridyl β-D-galactopyranoside (1h)

Using 4.8g of (R)-(+)-5-(1-methyl-2-pyrrolidinyl)-2-pyridyl 2,3,4,6-tetra-O-acetyl-β-D-galactopyranoside (5h),
(R)-(+)-5-(1-methyl-2-pyrrolidinyl)-2-pyridyl β-D-glucopyranoside (1h)(3.0g) was obtained in white solid form in the same manner as in 2 of Example 1.

Physical properties

$[\alpha]_D$ +40.6° (c 0.5, methanol)

IR: ν ($cm^{-1}$); 3356, 2950, 1607, 1491, 1406, 1340, 1253 1075

$^1$H - NMR: δ (CDCℓ$_3$; ppm from TMS)
1.80 - 2.10 (4H, m),
2.23 (3H, s),
2.25 (1H, m),
2.47 (1H, q, J = 9.4 Hz),
3.30 (2H, m),
3.62 (1H, dd, J = 3.3, 9.7 Hz),
3.73 (3H, m),
3.85 (1H, dd, J = 8.0, 9.7 Hz)
3.94 (1H, d, J = 3.3 Hz),
5.63 (1H, d, J = 8.0, Hz),
6.96 (1H, d, J = 8.6 Hz),
7.79 (1H, dd, J = 2.4, 8.6 Hz),
8.10 (1H, d, J = 2.4 Hz)

$^{13}$C-NMR: δ (CDCℓ$_3$; ppm from TMS);
22.8 ($CH_2$), 34.6 ($CH_2$) 40.2 ($CH_3$), 57.6 ($CH_2$), 62.2 ($CH_2$), 69.6 (CH), 70.0 (CH), 71.7 (CH), 74.8 (CH), 76.9 (CH), 98.8 (CH), 112.6 (CH), 132.0 (C), 140.1 (CH), 147.4 (CH), 163.8 (C)

MS (%): 340 ($M^+$) (3), 180 (12), 179 (17), 178 (72), 177 (71), 150 (29), 149 (90), 135 (25), 84 (100)

In the above physical properties, since disappearance of carbonyl absorption (in the vicinity of 1661 $cm^{-1}$) of the compound (4d) in IR, and a characteristic signal of β-O-glycoside (δ 5.63 (1H, d, J = 8.0 Hz)) in $^1$H - NMR are observed, the structure of the compound (1h) can be confirmed.

Example 9

Flavor and taste improving agent

The compounds (1a) to (1h) of the present invention, prepared in previous Examples were put to the following tests to evaluate their usefulness as a flavor and taste improving agent and a tobacco flavor.

1) The effect of the compounds (1a) to (1h) of the present invention on flavor, taste and stimulation

Ten percent ethanol solutions of the compounds (1a) to (1h) of the present invention were prepared. The solutions were spray-added to 100g of shredded tobacco leaf fillings of "MILD SEVEN" (trade name) (registered trademark). The tobacco fillings were rolled up and each was compared in terms of flavor, taste, and stimulation by a discrimination method with an unsprayed tobacco filling used as a control. The test was performed by 20 especially-trained special panelists.

The results were as shown in the following Tables 1 to 3.

Hereinafter, figures in tables in Tables of the specification indicate the number of people who evaluate good and "*" mark denotes the presence of a significant difference at 5% danger ratio.

Table 1

| Comparison results of flavor | | |
|---|---|---|
| Compound | Flavored Product | Non flavored product (control) |
| 1a | 15* | 5 |
| 1b | 16* | 4 |
| 1c | 15* | 5 |
| 1d | 17* | 3 |
| 1e | 16* | 4 |
| 1f | 18* | 2 |
| 1g | 16* | 4 |
| 1h | 17* | 3 |

Table 2

| Comparison results of taste | | |
|---|---|---|
| Compound | Flavored Product | Non flavored product (control) |
| 1a | 18* | 2 |
| 1b | 19* | 1 |
| 1c | 18* | 2 |
| 1d | 18* | 2 |
| 1e | 18* | 2 |
| 1f | 18* | 2 |
| 1g | 18* | 2 |
| 1h | 17* | 3 |

Table 3

| Comparison results of stimulation | | |
|---|---|---|
| Compound | Flavored Product | Non flavored product (control) |
| 1a | 12 | 8 |
| 1b | 11 | 9 |
| 1c | 12 | 8 |
| 1d | 12 | 8 |
| 1e | 11 | 9 |
| 1f | 12 | 8 |
| 1g | 13 | 7 |
| 1h | 13 | 7 |

As is apparent from Tables 1 to 3, Remarkable improvements in flavor, taste, and stimulation were achieved by addition of the compounds (1a) to (1h) of the present invention.

2) The effect of the compounds (1a) and (1h) of the present invention on flavor and taste

The compounds (1a) to (1h) of the present invention were classified into combinations: (1a) and (1b); (1c) and (1d); (1e) and (1f); and (1g) and (1h), and effects on the improving flavor and taste were tested.

An ethanol solution of the mixture containing compounds (1a) and (1b) of the present invention in a ratio of 1 : 1 was prepared. The solution was spray-added to 100g of a shredded tobacco leaf filling for "CABIN"(trade name) (registered trademark) such that it was contained in the shredded tobacco leaf filling in an amount of 0.001% (w/w) by weight. The tobacco filling was rolled up and compared with an unsprayed tobacco filling used as a control in terms of flavor and taste at the time of smoking by a discrimination method. The test was performed by 100 people of "CABIN" lovers.

The effects of other combinations on the improvement of flavor and taste were evaluated similarly.

The results were as shown in the following Table 4.

Table 4

| Improvement in flavor and taste | | |
|---|---|---|
| Combination of compounds | Flavored product | Non flavored product (control) |
| (1a) and (1b) | 81* | 19 |
| (1c) and (1d) | 82* | 18 |
| (1e) and (1f) | 84* | 16 |
| (1g) and (1h) | 84* | 16 |

As is apparent from Table 4, remarkable improvements on the flavor and taste were attained by addition of the compounds of the present invention.

The compounds of the present invention can be added by methods other than the aforementioned one. To be more specific, the compounds (1a) to (1h) may be used alone or in appropriate mixed form. Further, the compounds (1a) to (1h) of the present invention can be added in an amount of approximately 0.0001 to 0.01% (w/w) based on a shredded tobacco filling for rolled-up tobacco or a shredded tobacco filling used for raw material of a tobacco product prior to being packed.

[Industrial Applicability]

From the foregoing, the compounds of the present invention are substances excellent as a flavor and taste improving agent and effective in imparting suitable tobacco flavor and taste to a shredded tobacco filling and delicate depth to smoke.

Further, since the compounds of the present invention have specific structures having glucose in a molecular, the following effects are expected.

More specifically, in recent years, pharmacological activities of compounds containing a glycosyl and applications of these compounds to pharmaceutical agents have been intensively and extensively studied, producing good results. Particularly, a number of attempts have been made to introduce a glycosyl to a highly toxic compound so as to mitigate the toxicity (for example, 5-fluorouracil riboside). Presumably, the compounds having a glycosyl in a molecule will become important in future. Since the compounds of the present invention contain nicotine which is physiologically active as an aglycone, their application to pharmaceutical agents is expected.

## Claims

1. A novel nicotine derivative represented by the following formula (1).

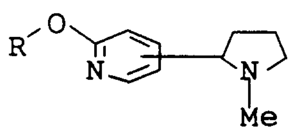

(1)

Wherein R represents a hexose or a dihexose Further,

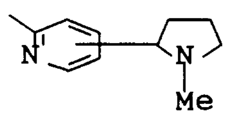

is an (S)-(-)-3-(1-methyl-2-pyrrolidinyl)-2-pyridyl group, an (R)-(+)-3-(1-methyl-2-pyrrolidinyl)-2-pyridyl group, an (S)-(-)-5-(1-methyl-2-pyrrolidinyl)-2-pyridyl group, or an (R)-(+)-5-(1-methyl-2-pyrrolidinyl)-2-pyridyl group.

2. A novel nicotine derivative according to claim 1, wherein R is one selected from the group consisting of glucose, galactose, mannose, glucosamine, galactosamine, mannosamine, lactose, and lactosamine.

3. A novel nicotine derivative according to claim 1, wherein R is one selected from the group consisting of glucose and galactose.

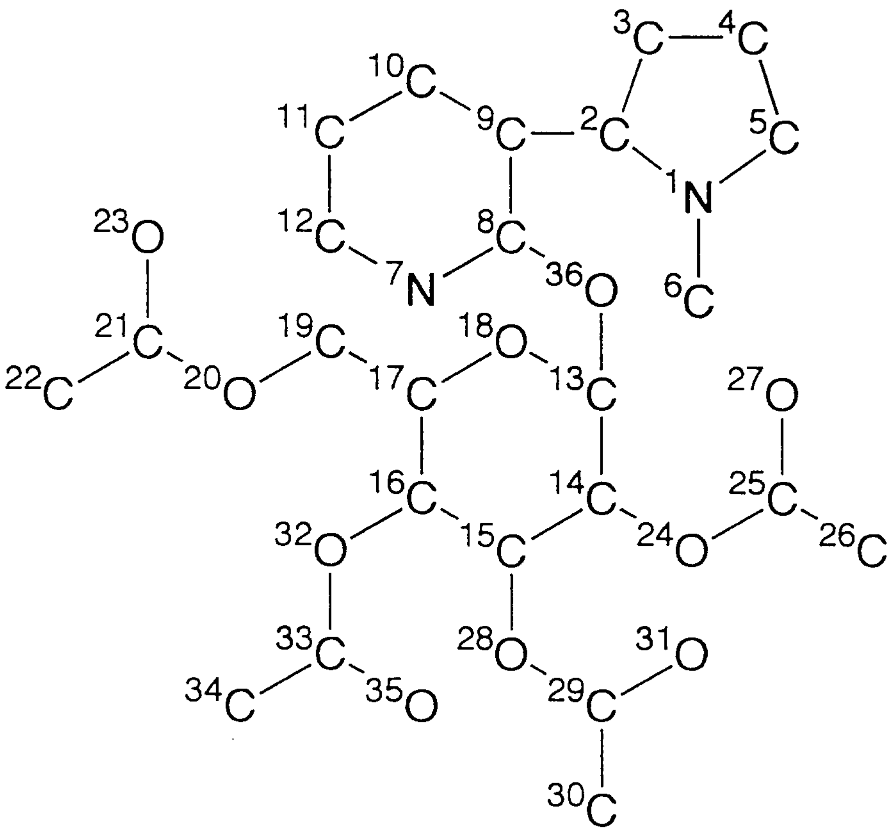

FIG. 1

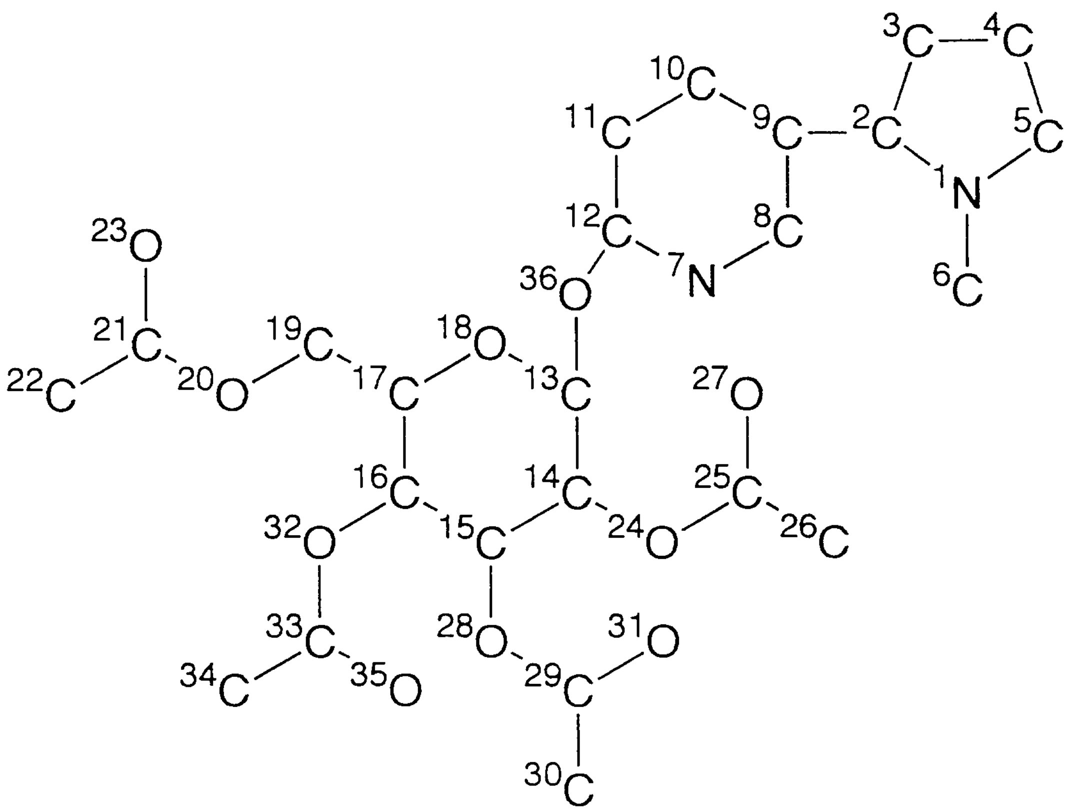

FIG. 2

**INTERNATIONAL SEARCH REPORT**

International application No. PCT/JP93/01151

**A. CLASSIFICATION OF SUBJECT MATTER**

Int. $Cl^5$ C07H17/02

According to International Patent Classification (IPC) or to both national classification and IPC

**B. FIELDS SEARCHED**

Minimum documentation searched (classification system followed by classification symbols)

Int. $Cl^5$ C07H17/02, C07D401/04, 401/14

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| A | Chemical Abstracts, Vol. 79, No. 23, p. 365 Abstract No. 136957f, December 10, 1973 (10. 12. 73), (Abstract on "Goldfarb, Ya. L. et al., "Synthesis of nitronicotines" Khim. Geterotsikl. Soedin, 1973. (8). p. 1062-6") | 1-3 |

☐ Further documents are listed in the continuation of Box C. ☐ See patent family annex.

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| November 1, 1993 (01. 11. 93) | November 22, 1993 (22. 11. 93) |
| Name and mailing address of the ISA/ Japanese Patent Office | Authorized officer |
| Facsimile No. | Telephone No. |